# EUROPEAN PATENT SPECIFICATION

(11) **EP 1 862 802 B1**
(45) Date of publication and mention of the grant of the patent: **10.12.2008**
(21) Application number: 06011409.7
(22) Date of filing: 01.06.2006
(51) Int. Cl.: G01N 33/574, C12Q 1/48, C07K 1/04, G01N 33/68

(54) **Methods for the identification of ZAP-70 interacting molecules and for the purification of ZAP-70**
Verfahren zur Identifizierung von mit ZAP-70 wechselwirkenden Molekülen und zur ZAP-70-Reinigung
Procédés d'identification des molécules d'interaction ZAP-70 et de purification de ZAP-70

(43) Date of publication of application: 05.12.2007
(73) Proprietor: CELLZOME AG, 69117 Heidelberg (DE)
(72) Inventor: Kruse, Ulrich, 69221 Dossenheim (DE); Ramsden, Nigel, Fowlmere, Royston, Herts SG8 7QP (GB); Drewes, Gerard, 69121 Heidelberg (DE); Eberhard, Dirk, 69295 Mauer (DE)
(74) Representative: Huhn, Michael

(56) References cited:
- EP-A- 1 662 259
- DATABASE BIOSIS [Online] BIOSCIENCES INFORMATION SERVICE, PHILADELPHIA, PA, US; November 2005 (2005-11), RASPADORI DONATELLA ET AL: "ZAP-70 expression in T cells of B-cell chronic lymphocytic leukaemia: Correlation with negative prognostic factors of the disease." XP002403587 Database accession no. PREV200600133407
- DATABASE BIOSIS [Online] BIOSCIENCES INFORMATION SERVICE, PHILADELPHIA, PA, US; November 2004 (2004-11), GUILLAUME NICOLAS ET AL: "ZAP-70 tyrosine kinase is constituvely expressed and phosphorylated in B-lineage acute lymphoblastic leukemia cells." XP002403588 Database accession no. PREV200510269869
- CHEN LIGUANG ET AL: "Expression of ZAP-70 is associated with increased B-cell receptor signaling in chronic lymphocytic leukemia." BLOOD, vol. 100, no. 13, 15 December 2002 (2002-12-15), pages 4609-4614, XP002403583 ISSN: 0006-4971
- ANNIS D ALLEN ET AL: "A general technique to rank protein-ligand binding affinities and determine allosteric versus direct binding site competition in compound mixtures." JOURNAL OF THE AMERICAN CHEMICAL SOCIETY. 1 DEC 2004, vol. 126, no. 47, 1 December 2004 (2004-12-01), pages 15495-15503, XP002403584 ISSN: 0002-7863
- WISNIEWSKI D ET AL: "CHARACTERIZATION OF POTENT INHIBITORS OF THE BER-ABL AND THE C-KIT RECEPTOR TYROSINE KINASES" CANCER RESEARCH, AMERICAN ASSOCIATION FOR CANCER RESEARCH, BALTIMORE, MD, US, vol. 62, no. 15, 1 August 2002 (2002-08-01), pages 4244-4255, XP001146932 ISSN: 0008-5472
- JIN LEI ET AL: "The three-dimensional structure of the ZAP-70 kinase domain in complex with staurosporine: implications for the design of selective inhibitors." THE JOURNAL OF BIOLOGICAL CHEMISTRY. 8 OCT 2004, vol. 279, no. 41, 8 October 2004 (2004-10-08), pages 42818-42825, XP002403594 ISSN: 0021-9258

### SEQUENCE LISTING

<110> CellZome AG
<120> Methods for the identification of ZAP-70 interacting molecules and for the purification of ZAP-70
<130> CEL64295EP
<160> 10
<170> PatentIn version 3.3
<210> 1
   <211> 12
   <212> PRT
   <213> Artificial
<220>
   <223> peptide fragment
<400> 1
   Lys Leu Pro Tyr Ser Gly Ala Gln Thr Asp Gly Lys
   1               5                   10
<210> 2
   <211> 12
   <212> PRT
   <213> artificial
<220>
   <223> peptide fragment
<400> 2
   Leu Lys Ala Asp Gly Leu Ile Pro Tyr Cys Leu Lys
   1               5                   10
<210> 3
   <211> 17
   <212> PRT
   <213> artificial
<220>
   <223> peptide fragment
<400> 3
   Arg Ile Asp Thr Leu Asn Ser Asp Gly Pro Tyr Thr Pro Glu Pro Ala
   1               5                   10                  15
   Arg
<210> 4
   <211> 16
   <212> PRT
   <213> artificial
<220>
   <223> peptide fragment
<400> 4
   Ile Asp Thr Leu Asn Ser Asp Gly Pro Tyr Thr Pro Glu Pro Ala Arg
   1               5                   10                  15
<210> 5
   <211> 9
   <212> PRT
   <213> artificial
<220>
   <223> peptide fragment
<400> 5
   Ile Thr Pro Ser Pro Asp Lys Pro Arg
   1               5
<210> 6
   <211> 13
   <212> PRT
   <213> artificial
<220>
   <223> peptide fragment
<400> 6
   Ala Leu Gly Ala Asp Asp Ser Pro Tyr Tyr Thr Ala Arg
   1               5                   10
<210> 7
   <211> 13
   <212> PRT
   <213> artificial
<220>
   <223> peptide fragment
<400> 7
   Ala Leu Gly Ala Asp Asp Ser Tyr Pro Tyr Thr Ala Arg
   1               5                   10
<210> 8
   <211> 14
   <212> PRT
   <213> peptide fragment
<400> 8
   Ala Leu Gly Ala Asp Asp Ser Pro Tyr Pro Tyr Thr Ala Arg
   1               5                   10
<210> 9
   <211> 10
   <212> PRT
   <213> artificial
<220>
   <223> peptide fragment
<400> 9
   Ala Cys Tyr Pro Tyr Ser Leu Ala Ser Lys
   1               5                   10
<210> 10
   <211> 628
   <212> PRT
   <213> homo sapiens
<400> 10
   Met Pro Asp Pro Ala Ala His Leu Pro Phe Phe Tyr Gly Ser Ile Ser
   1               5                   10                  15
   Arg Ala Glu Ala Glu Glu His Leu Lys Leu Ala Gly Met Ala Asp Gly
               20                  25                  30
   Leu Phe Leu Leu Arg Gln Cys Leu Arg Ser Leu Gly Gly Tyr Val Leu
           35                  40                  45
   Ser Leu Val His Asp Val Arg Phe His His Phe Pro Ile Glu Arg Gln
       50                  55                  60
   Leu Asn Gly Thr Tyr Ala Ile Ala Gly Gly Lys Ala His Cys Gly Pro
   65                  70                  75                  80
   Ala Glu Leu Cys Glu Phe Tyr Ser Arg Asp Pro Asp Gly Leu Pro Cys
                   85                  90                  95
   Asn Leu Arg Lys Pro Cys Asn Arg Pro Ser Gly Leu Glu Pro Gln Pro
               100                 105                 110
   Gly Val Phe Asp Cys Leu Arg Asp Ala Met Val Arg Asp Tyr Val Arg
           115                 120                 125
   Gln Thr Trp Lys Leu Glu Gly Glu Ala Leu Glu Gln Ala Ile Ile Ser
       130                 135                 140
   Gln Ala Pro Gln Val Glu Lys Leu Ile Ala Thr Thr Ala His Glu Arg
   145                 150                 155                 160
   Met Pro Trp Tyr His Ser Ser Leu Thr Arg Glu Glu Ala Glu Arg Lys
                   165                 170                 175
   Leu Tyr Ser Gly Ala Gln Thr Asp Gly Lys Phe Leu Leu Arg Pro Arg
               180                 185                 190
   Lys Glu Gln Gly Thr Tyr Ala Leu Ser Leu Ile Tyr Gly Lys Thr Val
           195                 200                 205
   Tyr His Tyr Leu Ile Ser Gln Asp Lys Ala Gly Lys Tyr Cys Ile Pro
       210                 215                 220
   Glu Gly Thr Lys Phe Asp Thr Leu Trp Gln Leu Val Glu Tyr Leu Lys
   225                 230                 235                 240
   Leu Lys Ala Asp Gly Leu Ile Tyr Cys Leu Lys Glu Ala Cys Pro Asn
   245 250 255
   Ser Ser Ala Ser Asn Ala Ser Gly Ala Ala Ala Pro Thr Leu Pro Ala
   260 265 270
   His Pro Ser Thr Leu Thr His Pro Gln Arg Arg Ile Asp Thr Leu Asn
   275 280 285
   Ser Asp Gly Tyr Thr Pro Glu Pro Ala Arg Ile Thr Ser Pro Asp Lys
   290 295 300
   Pro Arg Pro Met Pro Met Asp Thr Ser Val Tyr Glu Ser Pro Tyr Ser
   305 310 315 320
   Asp Pro Glu Glu Leu Lys Asp Lys Lys Leu Phe Leu Lys Arg Asp Asn
   325 330 335
   Leu Leu Ile Ala Asp Ile Glu Leu Gly Cys Gly Asn Phe Gly Ser Val
   340 345 350
   Arg Gln Gly Val Tyr Arg Met Arg Lys Lys Gln Ile Asp Val Ala Ile
   355 360 365
   Lys Val Leu Lys Gln Gly Thr Glu Lys Ala Asp Thr Glu Glu Met Met
   370 375 380
   Arg Glu Ala Gln Ile Met His Gln Leu Asp Asn Pro Tyr Ile Val Arg
   385 390 395 400
   Leu Ile Gly Val Cys Gln Ala Glu Ala Leu Met Leu Val Met Glu Met
   405 410 415
   Ala Gly Gly Gly Pro Leu His Lys Phe Leu Val Gly Lys Arg Glu Glu
   420 425 430
   Ile Pro Val Ser Asn Val Ala Glu Leu Leu His Gln Val Ser Met Gly
   435 440 445
   Met Lys Tyr Leu Glu Glu Lys Asn Phe Val His Arg Asp Leu Ala Ala
   450 455 460
   Arg Asn Val Leu Leu Val Asn Arg His Tyr Ala Lys Ile Ser Asp Phe
   465 470 475 480
   Gly Leu Ser Lys Ala Leu Gly Ala Asp Asp Ser Tyr Tyr Thr Ala Arg
   485 490 495
   Ser Ala Gly Lys Trp Pro Leu Lys Trp Tyr Ala Pro Glu Cys Ile Asn
   500 505 510
   Phe Arg Lys Phe Ser Ser Arg Ser Asp Val Trp Ser Tyr Gly Val Thr
   515 520 525
   Met Trp Glu Ala Leu Ser Tyr Gly Gln Lys Pro Tyr Lys Ala Gly Ala
   530 535 540
   Gly Arg Gly Arg Trp Ala Lys Met Lys Gly Pro Glu Val Met Ala Phe
   545 550 555 560
   Ile Glu Gln Gly Lys Arg Met Glu Cys Pro Pro Glu Cys Pro Pro Glu
   565 570 575
   Leu Tyr Ala Leu Met Ser Asp Cys Trp Ile Tyr Lys Trp Glu Asp Arg
   580 585 590
   Pro Asp Phe Leu Thr Val Glu Gln Arg Met Arg Ala Cys Tyr Tyr Ser
   595 600 605
   Leu Ala Ser Lys Val Glu Gly Pro Pro Gly Ser Thr Gln Lys Ala Glu
   610 615 620
   Ala Ala Cys Ala
   625

### SEQUENCE LISTING

<110> Cellzome AG
<120> Methods for the identification of ZAP-70 interacting molecules and for the purification of ZAP-70
<130> CEL64295EP
<160> 10
<170> PatentIn version 3.3
<210> 1
   <211> 12
   <212> PRT
   <213> Artificial
<220>
   <223> peptide fragment
<400> 1
   Lys Leu Pro Tyr Ser Gly Ala Gln Thr Asp Gly Lys
   1 5 10
<210> 2
   <211> 12
   <212> PRT
   <213> artificial
<220>
   <223> peptide fragment
<400> 2
   Leu Lys Ala Asp Gly Leu Ile Pro Tyr Cys Leu Lys
   1 5 10
<210> 3
   <211> 17
   <212> PRT
   <213> artificial
<220>
   <223> peptide fragment
<400> 3
   Arg Ile Asp Thr Leu Asn Ser Asp Gly Pro Tyr Thr Pro Glu Pro Ala
   1 5 10 15
   Arg
<210> 4
   <211> 16
   <212> PRT
   <213> artificial
<220>
   <223> peptide fragment
<400> 4
   Ile Asp Thr Leu Asn Ser Asp Gly Pro Tyr Thr Pro Glu Pro Ala Arg
   1 5 10 15
<210> 5
   <211> 9
   <212> PRT
   <213> artificial
<220>
   <223> peptide fragment
<400> 5
   Ile Thr Pro Ser Pro Asp Lys Pro Arg
   1 5
<210> 6
   <211> 13
   <212> PRT
   <213> artificial
<220>
   <223> peptide fragment
<400> 6
   Ala Leu Gly Ala Asp Asp Ser Pro Tyr Tyr Thr Ala Arg
   1 5 10
<210> 7
   <211> 13
   <212> PRT
   <213> artificial
<220>
   <223> peptide fragment
<400> 7
   Ala Leu Gly Ala Asp Asp Ser Tyr Pro Tyr Thr Ala Arg
   1 5 10
<210> 8
   <211> 14
   <212> PRT
   <213> peptide fragment
<400> 8
   Ala Leu Gly Ala Asp Asp Ser Pro Tyr Pro Tyr Thr Ala Arg
   1 5 10
<210> 9
   <211> 10
   <212> PRT
   <213> artificial
<220>
   <223> peptide fragment
<400> 9
   Ala Cys Tyr Pro Tyr Ser Leu Ala Ser Lys
   1 5 10
<210> 10
   <211> 628
   <212> PRT
   <213> homo sapiens
<400> 10
   Met Pro Asp Pro Ala Ala His Leu Pro Phe Phe Tyr Gly Ser Ile Ser
   1               5                   10                  15
   Arg Ala Glu Ala Glu Glu His Leu Lys Leu Ala Gly Met Ala Asp Gly
               20                  25                  30
   Leu Phe Leu Leu Arg Gln Cys Leu Arg Ser Leu Gly Gly Tyr Val Leu
           35                  40                  45
   Ser Leu Val His Asp Val Arg Phe His His Phe Pro Ile Glu Arg Gln
       50                  55                  60
   Leu Asn Gly Thr Tyr Ala Ile Ala Gly Gly Lys Ala His Cys Gly Pro
   65                  70                  75                  80
   Ala Glu Leu Cys Glu Phe Tyr Ser Arg Asp Pro Asp Gly Leu Pro Cys
                   85                  90                  95
   Asn Leu Arg Lys Pro Cys Asn Arg Pro Ser Gly Leu Glu Pro Gln Pro
               100                 105                 110
   Gly Val Phe Asp Cys Leu Arg Asp Ala Met Val Arg Asp Tyr Val Arg
           115                 120                 125
   Gln Thr Trp Lys Leu Glu Gly Glu Ala Leu Glu Gln Ala Ile Ile Ser
       130                 135                 140
   Gln Ala Pro Gln Val Glu Lys Leu Ile Ala Thr Thr Ala His Glu Arg
   145                 150                 155                 160
   Met Pro Trp Tyr His Ser Ser Leu Thr Arg Glu Glu Ala Glu Arg Lys
                   165                 170                 175
   Leu Tyr Ser Gly Ala Gln Thr Asp Gly Lys Phe Leu Leu Arg Pro Arg
               180                 185                 190
   Lys Glu Gln Gly Thr Tyr Ala Leu Ser Leu Ile Tyr Gly Lys Thr Val
           195                 200                 205
   Tyr His Tyr Leu Ile Ser Gln Asp Lys Ala Gly Lys Tyr Cys Ile Pro
       210                 215                 220
   Glu Gly Thr Lys Phe Asp Thr Leu Trp Gln Leu Val Glu Tyr Leu Lys
   225                 230                 235                 240
   Leu Lys Ala Asp Gly Leu Ile Tyr Cys Leu Lys Glu Ala Cys Pro Asn
                   245                 250                 255
   Ser Ser Ala Ser Asn Ala Ser Gly Ala Ala Ala Pro Thr Leu Pro Ala
               260                 265                 270
   His Pro Ser Thr Leu Thr His Pro Gln Arg Arg Ile Asp Thr Leu Asn
           275                 280                 285
   Ser Asp Gly Tyr Thr Pro Glu Pro Ala Arg Ile Thr Ser Pro Asp Lys
       290                 295                 300
   Pro Arg Pro Met Pro Met Asp Thr Ser Val Tyr Glu Ser Pro Tyr Ser
   305                 310                 315                 320
   Asp Pro Glu Glu Leu Lys Asp Lys Lys Leu Phe Leu Lys Arg Asp Asn
                   325                 330                 335
   Leu Leu Ile Ala Asp Ile Glu Leu Gly Cys Gly Asn Phe Gly Ser Val
               340                 345                 350
   Arg Gln Gly Val Tyr Arg Met Arg Lys Lys Gln Ile Asp Val Ala Ile
           355                 360                 365
   Lys Val Leu Lys Gln Gly Thr Glu Lys Ala Asp Thr Glu Glu Met Met
       370                 375                 380
   Arg Glu Ala Gln Ile Met His Gln Leu Asp Asn Pro Tyr Ile Val Arg
   385                 390                 395                 400
   Leu Ile Gly Val Cys Gln Ala Glu Ala Leu Met Leu Val Met Glu Met
                   405                 410                 415
   Ala Gly Gly Gly Pro Leu His Lys Phe Leu Val Gly Lys Arg Glu Glu
               420                 425                 430
   Ile Pro Val Ser Asn Val Ala Glu Leu Leu His Gln Val Ser Met Gly
           435                 440                 445
   Met Lys Tyr Leu Glu Glu Lys Asn Phe Val His Arg Asp Leu Ala Ala
       450                 455                 460
   Arg Asn Val Leu Leu Val Asn Arg His Tyr Ala Lys Ile Ser Asp Phe
   465                 470                 475                 480
   Gly Leu Ser Lys Ala Leu Gly Ala Asp Asp Ser Tyr Tyr Thr Ala Arg
                   485                 490                 495
   Ser Ala Gly Lys Trp Pro Leu Lys Trp Tyr Ala Pro Glu Cys Ile Asn
               500                 505                 510
   Phe Arg Lys Phe Ser Ser Arg Ser Asp Val Trp Ser Tyr Gly Val Thr
           515                 520                 525
   Met Trp Glu Ala Leu Ser Tyr Gly Gln Lys Pro Tyr Lys Ala Gly Ala
       530                 535                 540
   Gly Arg Gly Arg Trp Ala Lys Met Lys Gly Pro Glu Val Met Ala Phe
   545                 550                 555                 560
   Ile Glu Gln Gly Lys Arg Met Glu Cys Pro Pro Glu Cys Pro Pro Glu
                   565                 570                 575
   Leu Tyr Ala Leu Met Ser Asp Cys Trp Ile Tyr Lys Trp Glu Asp Arg
               580                 585                 590
   Pro Asp Phe Leu Thr Val Glu Gln Arg Met Arg Ala Cys Tyr Tyr Ser
           595                 600                 605
   Leu Ala Ser Lys Val Glu Gly Pro Pro Gly Ser Thr Gln Lys Ala Glu
       610                 615                 620
   Ala Ala Cys Ala
   625

## Description

The present invention relates to methods for the identification of ZAP-70 interacting molecules and for the purification of ZAP-70 using aminopyrido-pyrimidine ligand 24 as a ligand for ZAP-70. Furthermore, the present invention relates to pharmaceutical compositions comprising said interacting molecules e.g. for the treatment of T-cell mediated diseases such as autoimmune disease, inflammation and transplant rejection.

Protein kinases participate in the signaling events which control the activation, growth and differentiation of cells in response to extracellular mediators or stimuli such as growth factors, cytokines or chemokines. In general, these kinases are classified in two groups, those that preferentially phosphorylate tyrosine residues and those that preferentially phosphorylate serine and/or threonine residues. The tyrosine kinases include membrane-spanning growth factor receptors such as the epidermal growth factor receptor (EGFR) and cytosolic non-receptor kinases such as Src, Syk or ZAP-70.

Inappropriately high protein kinase activity is involved in many diseases including cancer and inflammatory disorders. This can be caused either directly or indirectly by the failure of control mechanisms due to mutation, overexpression or inappropriate activation of the enzyme. In all of these instances, selective inhibition of the kinase is expected to have a beneficial effect.

Protein tyrosine kinases - both receptor tyrosine kinases and non-receptor kinases - are essential for the activation and proliferation of cells of the immune system. Among the earliest detectable events upon the immunoreceptor activation in mast cells, T cells and B cells is the stimulation of non-receptor tyrosine kinases. Immune receptors such as the high-affinity IgE receptor (FcεRI), T cell antigen receptor (TCR) and B cell receptor, consist of antigen-binding subunits and signal transducing subunits. The signal transducing chain contains one or more copies of immunoreceptor tyrosine-based activation motifs (ITAMSs). For TCR activation, ITAMS located in the CD3 molecule are phosphorylated by Lck and Fyn, two Src family tyrosine kinases, followed by recruitment and activation of ZAP-70, a member of the Syk family of tyrosine kinases. These activated tyrosine kinases then phosphorylate downstream adaptor molecules such as LAT (linker for activation of T cells) and SLP-76 (SH2 domain-containing leukocyte protein of 76 kDa). This step leads to the activation of multiple downstream signaling molecules such as inducible T cell kinase (ITK), PLCγ1 and PI3 kinase (Wong, 2005, Current Opinion in Pharmacology 5, 1-8).

ZAP-70 (zeta-associated protein of 70 kDa) belongs to the Syk family of tyrosine kinases and is associated with the zeta subunit of the T cell receptor (Chan et al., 1991, Proc. Natl. Acad. Sci. USA 88, 9166-9170; Weiss, 1993, Cell 73, 209-212). ZAP-70 is primarily expressed in T cells and Natural Killer (NK) cells and plays an essential role in signaling through the TCR. The TCR-mediated activation of T cells is crucial for the immune response. Failure to adequately regulate T cell activation can lead to allergic and autoimmune diseases. Therefore ZAP-70 is considered as an attractive target for the development of immunosuppresive agents for T cell mediated diseases.

Several approaches for the identification of selective ZAP-70 inhibitors have been reported. Vu suggested the structure-based design and synthesis of antagonists of the tandem Src-homology 2 (SH2) domains of ZAP-70 (Vu, 2000, Curr. Med. Chem. 7(10), 1081-1100). Nishikawa screened a peptide library for the ability to bind to ZAP-70 and identified a peptide that inhibited ZAP-kinase activity by competing with protein substrates (Nishikawa et al., 2000, Molecular Cell 6, 969-974). Moffat used a ZAP-70 kinase assay with the non-physiological substrate polyGluTyr to identify ZAP-70 inhibitors (Moffat et al., 1999, Bioorg. Med. Chem. Letters 9, 3351-3356). In addition, the three-dimensional structure of the ZAP-70 kinase domain in complex with Staurosporine was reported and suggested as basis for the structure-based design of inhibitors (Jin et al., 2004, J. Biol. Chem. 279(41), 42818-42825).

Annis, D. A., J. Am. Chem. Soc. 2004, 126, 15495-15503 describe affinity selection-mass spectrometry techniques for protein ligand high-throughput screening for ZAP-70 using competitive binding of several ligands.

Although ZAP-70 and its role in TCR signaling was already discovered in 1991 (Chan et al., 1991, Proc. Natl. Acad. Sci. USA 88, 9166-9170) and soon afterwards recognized as a logical drug target for T cell mediated diseases, no ZAP-70 inhibitors have been approved as drugs yet. One reason for the failure to identify and develop selective ZAP-70 inhibitors is the lack of effective assay methods to identify compounds that interact with the physiological form of ZAP-70 as it occurs in activated T-cells.

In view of the above, there is a need for providing effective methods for the identification of ZAP-70 interacting compounds as well as for methods for the purification of ZAP-70.

To comply with this need, the invention provides in a first aspect a method for the identification of a ZAP-70 interacting compound, comprising the steps of
a) providing a protein preparation containing phosphorylated ZAP-70,
b) contacting the protein preparation with 7-(4-Aminomethyl-phenylamino)-3-(2,6-dichloro-phenyl)-1-methyl-1H-[1,6]naphthyridin-2-one immobilized on a solid support under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex,
c) incubating the 7-(4-Aminomethyl-phenylamino)-3-(2,6-dichloro-phenyl)-1-methyl-1H-[1,6]naphthyridin-2-one - phosphorylated ZAP-70 complex with a given compound, and
d) determining whether the compound is able to separate phosphorylated ZAP-70 from the immobilized 7-(4-Aminomethyl-phenylamino)-3-(2,6-dichloro-phenyl)-1-methyl-1H-[1,6]naphthyridin-2-one.

In a second aspect, the present invention relates to a method for the identification of a ZAP-70 interacting compound, comprising the steps of
a) providing a protein preparation containing phosphorylated ZAP-70,
b) contacting the protein preparation with 7-(4-Aminomethyl-phenylamino)-3-(2,6-dichloro-phenyl)-1-methyl-1H-[1,6]naphthyridin-2-one immobilized on a solid support and with a given compound under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex, and
c) detecting the 7-(4-Aminomethyl-phenylamino)-3-(2,6-dichloro-phenyl)-1-methyl-1H-[1,6]naphthyridin-2-one - phosphorylated ZAP-70 complex formed in step b).

In a third aspect, the invention provides a method for the identification of a ZAP-70 interacting compound, comprising the steps of:
a) providing two aliquots of a protein preparation containing phosphorylated ZAP-70,
b) contacting one aliquot with 7-(4-Aminomethyl-phenylamino)-3-(2,6-dichloro-phenyl)-1-methyl-1H-[1,6]naphthyridin-2-one immobilized on a solid support under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex,
c) contacting the other aliquot with 7-(4-Aminomethyl-phenylamino)-3-(2,6-dichloro-phenyl)-1-methyl-1H-[1,6]naphthyridin-2-one immobilized on a solid support and with a given compound under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex, and
d) determining the amount of 7-(4-Aminomethyl-phenylamino)-3-(2,6-dichloro-phenyl)-1-methyl-1H-[1,6]naphthyridin-2-one - phosphorylated ZAP-70 complex formed in steps b) and c).

In a fourth aspect, the invention relates to a method for the identification of a ZAP-70 interacting compound, comprising the steps of:
a) providing two aliquots comprising each at least one cell containing phosphorylated ZAP-70,
b) incubating one aliquot with a given compound,
c) harvesting the cells of each aliquot,
d) lysing the cells in order to obtain protein preparations,
e) contacting the protein preparations with 7-(4-Aminomethyl-phenylamino)-3-(2,6-dichloro-phenyl)-1-methyl-1H-[1,6]naphthyridin-2-one immobilized on a solid support under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex, and
f) determining the amount of 7-(4-Aminomethyl-phenylamino)-3-(2,6-dichloro-phenyl)-1-methyl-1H-[1,6]naphthyridin-2-one - phosphorylated ZAP-70 complex formed in each aliquot in step e).

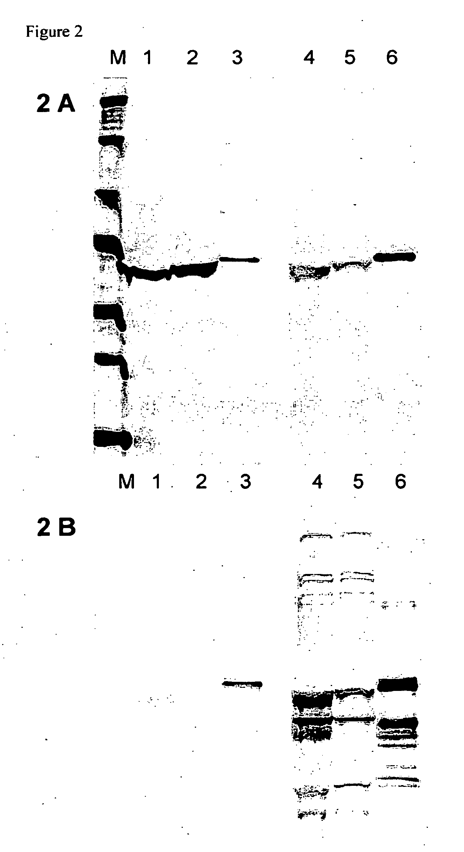

In the context of the present invention, it has been surprisingly found that 7-(4-Aminomethyl-phenylamino)-3-(2,6-dichloro-phenyl)-1-methyl-1H-[1,6]naphthyridin-2-one is a ZAP-70 ligand which recognizes preferably phosphorylated ZAP-70 (see Figure 2). This enables the use of 7-(4-Aminomethyl-phenylamino)-3-(2,6-dichloro-phenyl)-1-methyl-1H-[1,6]naphthyridin-2-one in screening assays, e.g. in competitive screening assays as well as in methods for the purification of phosphorylated ZAP-70.

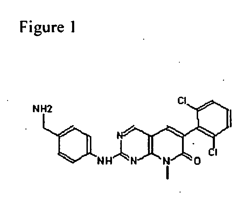

The structure of 7-(4-Aminomethyl-phenylamino)-3-(2,6-dichloro-phenyl)-1-methyl-1H-[1,6]naphthyridin-2-one also (aminopyrido-pyrimidine ligand 24) is given in Fig. 1. This compound is a substituted aminopyrido-pyrimidine compound. 7-(4-Aminomethyl-phenylamino)-3-(2,6-dichloro-phenyl)-1-methyl-1H-[1,6]naphthyridin-2-one can be covalently coupled to a suitable solid support material via the primary amino group and be used for the isolation of binding proteins. The synthesis of 7-(4-Aminomethyl-phenylamino)-3-(2,6-dichloro-phenyl)-1-methyl-1H-[1,6]naphthyridin-2-one is described in Example 1. According to the invention, the expression "7-(4-Aminomethyl-phenylamino)-3-(2,6-dichloro-phenyl)-1-methyl-1H-[1,6]naphthyridin-2-one" also includes compounds comprising the identical core but which have another linker, preferably coupled to the amino group not being part of the cyclic structures, for linkage to the solid support. Typically linkers have backbone of 8, 9 or 10 atoms. The linkers may contain either a carboxy-, hydroxy or amino-active group.

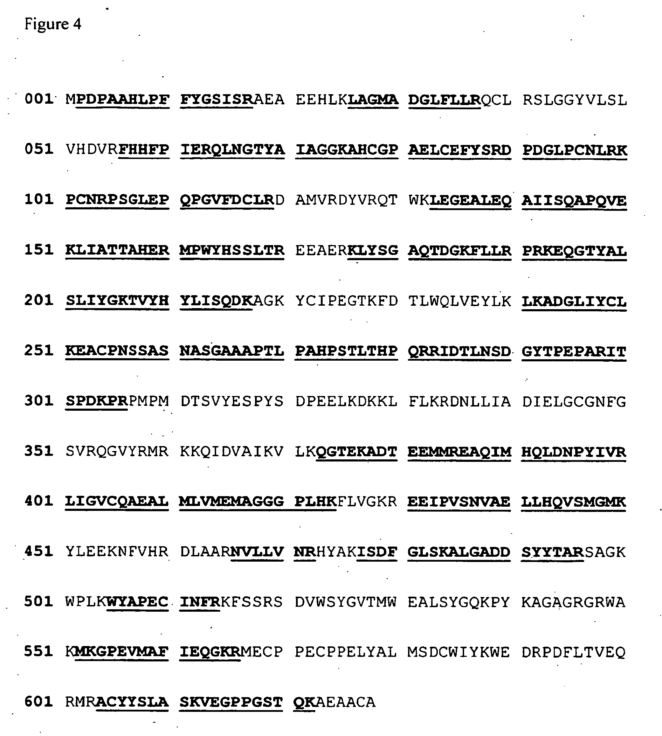

According to the present invention, the expression "ZAP-70" does not only mean the human protein as shown in Figure 4 but also a functionally active derivative thereof, or a functionally active fragment thereof, or a homologue thereof, or a variant encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

In some aspects of the invention, first a protein preparation containing phosphorylated ZAP-70 is provided. The methods of the present invention can be performed with any protein preparation as a starting material, as long as the phosphorylated ZAP-70 is solubilized in the preparation. Examples include a liquid mixture of several proteins, a cell lysate, a partial cell lysate which contains not all proteins present in the original cell or a combination of several cell lysates.

The presence of phosphorylated ZAP-70 protein species in a protein preparation of interest can be detected on Western blots probed with antibodies that are specifically directed against ZAP-70 phopshorylation sites, for example phosphorylated Tyrosine residues at position 126, 292, 319, 492 or 493 (Watts et al., 1994, The Journal of Biological Chemistry 269(4), 29520-29529). Antibodies directed against phosporylated Serine or threonine can also be used. Such phospho-specific anti-ZAP-70 antibodies can be obtained from commercial suppliers (e.g. Upstate or Cell Signaling). The use of such anti-phospho antibodies in conjunction with Western blot analysis to detect phosphorylated ZAP-70 has been described (Houtman et al., 2005, The Journal of Immunology-175(4), 2449-2458).

Cell lysates or partial cell lysates can be obtained by isolating cell organelles (e.g. nucleus, mitochondria, ribosomes, golgi etc.) first and then preparing protein preparations derived from these organelles. Methods for the isolation of cell organelles are known in the art (Chapter 4.2 Purification of Organelles from Mammalian Cells in ""Current Protocols in Protein Science", Editors: John.E. Coligan, Ben M. Dunn, Hidde L. Ploegh, David W. Speicher, Paul T. Wingfield; Wiley, ISBN: 0-471-14098-8).

In addition, protein preparations can be prepared by fractionation of cell extracts thereby enriching specific types of proteins such as cytoplasmic or membrane proteins (Chapter 4.3 Subcellular Fractionation of Tissue Culture Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

Furthermore protein preparations from body fluids can be used (e.g. blood, cerebrospinal fluid, peritoneal fluid and urine).

For example whole embryo lysates derived from defined development stages or adult stages of model organisms such as C. elegans can be used. In addition, whole organs such as heart dissected from mice can be the source of protein preparations. These organs can also be perfused in vitro in order to obtain a protein preparation.

Furthermore, the protein preparation may be a preparation containing phosphorylated ZAP-70 which has been recombinantely produced. Methods for the production of recombinant proteins in prokaryotic and eukaryotic cells are widely established (Chapter 5 Production of Recombinant Proteins in "Current Protocols in Protein Science", Editors: John. E. Coligan, Ben M. Dunn, Hidde L. Ploegh, David W. Speicher, Paul T. Wingfield; Wiley, 1995, ISBN: 0-471-14098-8).

In a preferred embodiment of the methods of the invention, the provision of a protein preparation includes the steps of harvesting at least one cell containing phosphorylated ZAP-70 and lysing the cell.

The ZAP-70 protein is preferentially expressed in T lymphocytes and Natural Killer (NK) cells. Therefore cells isolated from peripheral blood represent a suitable biological material. Procedures for the preparation and culture of human lymphocytes and lymphocyte subpopulations obtained from peripheral blood (PBLs) are widely known (W.E Biddison, Chapter 2.2 "Preparation and culture of human lymphocytes" in Current Protocols in Cell Biology, 1998, John Wiley & Sons, Inc.). For example, density gradient centrifugation is a method for the separation of lymphocytes from other blood cell populations (e.g. erythrocytes and granulocytes). Human lymphocyte subpopulations can be isolated via their specific cell surface receptors which can be recognized by monoclonal antibodies. The physical separation method involves coupling of these antibody reagents to magnetic beads which allow the enrichment of cells that are bound by these antibodies (positive selection). The isolated lymphocyte cells can be further cultured and stimulated by adding antibodies directed against the T-cell receptor or co-receptors such as CD-3 to initiate T-cell recptor signaling and subsequently phosphorylation of ZAP-70 (Houtman et al., 2005, The Journal of Immunology 175(4), 2449-2458).

As an alternative to primary human cells cultured cell lines (e.g. Jurkat cells) can be used and stimulated in analogous fashion with anti-CD3 antibodies to obtain phosphorylated ZAP-70 (Kim and White, 2006, The Journal of Immunology 176(5):2833-2843).

Alternatively, it is also possible to incubate said lymphocytes or cell lines with phosphatase inhibitors in order to keep ZAP-70 in its phosphorylated state. Such methods are known in the art (D.C. Weiser and S. Shenolikar, Chapter 18.10 in Current Protocols in Molecular Biology, 2003, John Wiley & Sons, Inc.).

In a preferred embodiment, the cell is part of a cell culture system and methods for the harvest of a cell out of a cell culture system are known in the art (literature supra).

The choice of the cell will mainly depend on the expression of ZAP-70, since it has to be ensured that the protein is principally present in the cell of choice. In order to determine whether a given cell is a suitable starting system for the methods of the invention, methods like Westernblot, PCR-based nucleic acids detection methods, Northernblots and DNA-microarray methods ("DNA chips") might be suitable in order to determine whether a given protein of interest is present in the cell.

The choice of the cell may also be influenced by the purpose of the study. If the in vivo efficacy for a given drug needs to be analyze then cells or tissues may be selected in which the desired therapeutic effect occurs (e.g. T cells, NK cells or ZAP-70 positive CLL cells). By contrast, for the elucidation of protein targets mediating unwanted side effects the cell or tissue may be analysed in which the side effect is observed (e.g. bone marrow, thymus).

Furthermore, it is envisaged within the present invention that the cell containing phosphorylated ZAP-70 may be obtained from an organism, e.g. by biopsy. Corresponding methods are known in the art. For example, a biopsy is a diagnostic procedure used to obtain a small amount of tissue, which can then be examined miscroscopically or with biochemical methods. Biopsies are important to diagnose, classify and stage a disease, but also to evaluate and monitor drug treatment.

It is encompassed within the present invention that by the harvest of the at least one cell, the lysis is performed simultaneously. However, it is equally preferred that the cell is first harvested and then separately lysed.

Methods for the lysis of cells are known in the art (Karwa and Mitra: Sample preparation for the extraction, isolation, and purification of Nuclei Acids; chapter 8 in "Sample Preparation Techniques in Analytical Chemistry", Wiley 2003, Editor: Somenath Mitra, print ISBN: 0471328456; online ISBN: 0471457817). Lysis of different cell types and tissues can be achieved by homogenizers (e.g. Potter-homogenizer), ultrasonic desintegrators, enzymatic lysis, detergents (e.g. NP-40, Triton X-100, CHAPS, SDS), osmotic shock, repeated freezing and thawing, or a combination of these methods.

According to the methods of the invention, the protein preparation containing phosphorylated ZAP-70 is contacted with the aminopyrido-pyrimidine ligand 24 immobilized on a solid support under conditions allowing the formation of a aminopyrido-pyrimidine ligand 24phosphorylated ZAP-70 complex.

In the present invention, the term "an aminopyrido-pyrimidine ligand 24-phosphorylated ZAP-70 complex" denotes a complex where aminopyrido-pyrimidine ligand 24 interacts with ZAP-70, e.g. by covalent or, most preferred, by non-covalent binding.

The skilled person will know which conditions can be applied in order to enable the formation of the aminopyrido-pyrimidine ligand 24-phosphorylated ZAP-70 complex.

In the context of the present invention, the term "under conditions allowing the formation of the complex" includes all conditions under which such formation, preferably such binding is possible. This includes the possibility of having the solid support on an immobilized phase and pouring the lysate onto it. In another preferred embodiment, it is also included that the solid support is in a particulate form and mixed with the cell lysate.

In the context of non-covalent binding, the binding between aminopyrido-pyrimidine ligand 24 and phosphorylated ZAP-70 is, e.g., via salt bridges, hydrogen bonds, hydrophobic interactions or a combination thereof.

In a preferred embodiment, the steps of the formation of the aminopyrido-pyrimidine ligand 24 - phosphorylated ZAP-70 complex are performed under essentially physiological conditions. The physical state of proteins within cells is described in Petty, 1998 (Howard R. Petty1, Chapter 1, Unit 1.5 in: Juan S. Bonifacino, Mary Dasso, Joe B. Harford, Jennifer Lippincott-Schwartz, and Kenneth M. Yamada (eds.) Current Protocols in Cell Biology Copyright © 2003 John Wiley & Sons, Inc. All rights reserved. DOI: 10.1002/0471143030.cb0101s00Online Posting Date: May, 2001Print Publication Date: October, 1998).

The contacting under essentially physiological conditions has the advantage that the interactions between the ligand, the cell preparation (i. e. the enzyme to be characterized) and optionally the compound reflect as much as possible the natural conditions. "Essentially physiological conditions" are *inter alia* those conditions which are present in the original, unprocessed sample material. They include the physiological protein concentration, pH, salt concentration, buffer capacity and post-translational modifications of the proteins involved. The term "essentially physiological conditions" does not require conditions identical to those in the original living organism, wherefrom the sample is derived, but essentially cell-like conditions or conditions close to cellular conditions. The person skilled in the art will, of course, realize that certain constraints may arise due to the experimental set-up which will eventually lead to less cell-like conditions. For example, the eventually necessary disruption of cell walls or cell membranes when taking and processing a sample from a living organism may require conditions which are not identical to the physiological conditions found in the organism. Suitable variations of physiological conditions for practicing the methods of the invention will be apparent to those skilled in the art and are encompassed by the term "essentially physiological conditions" as used herein. In summary, it is to be understood that the term "essentially physiological conditions" relates to conditions close to physiological conditions, as e. g. found in natural cells, but does not necessarily require that these conditions are identical.

For example, "essentially physiological conditions" may comprise 50-200 mM NaCl or KCl, pH 6.5-8.5, 20-45°C, and 0.001-10 mM divalent cation (e.g. Mg++, Ca++,); more preferably about 150 m NaCl or KCl, pH7.2 to 7.6, 5 mM divalent cation and often include 0.01-1.0 percent non-specific protein (e.g. BSA). A non-ionic detergent (Tween, NP-40, Triton-X100) can often be present, usually at about 0.001 to 2%, typically 0.05-0.2% (volume/volume). For general guidance, the following buffered aequous conditions may be applicable: 10-250 mM NaCl, 5-50 mM Tris HCl, pH5-8, with optional addition of divalent cation(s) and/or metal chelators and/or non-ionic detergents.

Preferably, "essentially physiological conditions" mean a pH of from 6.5 to 7.5, preferably from 7.0 to 7.5, and / or a buffer concentration of from 10 to 50 mM, preferably from 25 to 50 mM, and / or a concentration of monovalent salts (e.g. Na or K) of from 120 to 170 mM, preferably 150 mM. Divalent salts (e.g. Mg or Ca) may further be present at a concentration of from 1 to 5 mM, preferably 1 to 2mM, wherein more preferably the buffer is selected from the group consisting of Tris-HCl or HEPES.

In the context of the present invention, aminopyrido-pyrimidine ligand 24 is immobilized on a solid support. Throughout the invention, the term "solid support" relates to every undissolved support being able to immobilize a small molecule ligand on its surface.

According to a further preferred embodiment, the solid support is selected from the group consisting of agarose, modified agarose, sepharose beads (e.g. NHS-activated sepharose), latex, cellulose, and ferro- or ferromagnetic particles.

Aminopyrido-pyrimidine ligand 24 may be coupled to the solid support either covalently or non-covalently. Non-covalent binding includes binding via biotin affinity ligands binding to steptavidin matrices.

Preferably, the aminopyrido-pyrimidine ligand 24 is covalently coupled to the solid support.

Before the coupling, the matrixes can contain active groups such as NHS, Carbodimide etc. to enable the coupling reaction with the aminopyrido-pyrimidine ligand 24. The aminopyrido-pyrimidine ligand 24 can be coupled to the solid support by direct coupling (e.g. using functional groups such as amino-, sulfhydryl-, carboxyl-, hydroxyl-, aldehyde-, and ketone groups) and by indirect coupling (e.g. via biotin, biotin being covalently attached to aminopyrido-pyrimidine ligand 24 and non-covalent binding of biotin to streptavidin which is bound to solid support directly).

The linkage to the solid support material may involve cleavable and non-cleavable linkers. The cleavage may be achieved by enzymatic cleavage or treatment with suitable chemical methods.

Preferred binding interfaces for binding aminopyrido-pyrimidine ligand 24 to solid support material are linkers with a C-atom backbone. Typically linkers have backbone of 8, 9 or 10 atoms. The linkers contain either a carboxy- or amino-active group.

The skilled person will appreciate that between the individual steps of the methods of the invention, washing steps may be necessary. Such washing is part of the knowledge of the person skilled in the art. The washing serves to remove non-bound components of the cell lysate from the solid support. Nonspecific (e.g. simple ionic) binding interactions can be minimized by adding low levels of detergent or by moderate adjustments to salt concentrations in the wash buffer.

According to the identification methods of the invention, the read-out sytem is either the detection or determination of phosphorylated ZAP-70 (first aspect of the invention), the detection of the aminopyrido-pyrimidine ligand 24-phosphorylated ZAP-70 complex (second aspect of the invention), or the determination of the amount of the aminopyrido-pyrimidine ligand 24-phosphorylated ZAP-70 complex (second, third and forth aspect of the invention).

The detection of the aminopyrido-pyrimidine ligand 24-phosphorylated ZAP-70 complex according to the second aspect of the invention can be performed by using labeled antibodies directed against phosphorylated ZAP-70 and a suitable readout system.

According to a preferred embodiment of the second aspect of the invention, the aminopyrido-pyrimidine ligand 24-phosphorylated ZAP-70 complex is detected by determining its amount.

In the course of the second, third and forth aspect of the invention, it is preferred that phosphorylated ZAP-70 is separated from the immobilized aminopyrido-pyrimidine ligand 24 in order to determine the amount of the aminopyrido-pyrimidine ligand 24-phosphorylated ZAP-70 complex.

According to invention, separating means every action which destroys the interactions between aminopyrido-pyrimidine ligand 24 and phosphorylated ZAP-70. This includes in a preferred embodiment the elution of phosphorylated ZAP-70 from the immobilized aminopyrido-pyrimidine ligand 24.

The elution can be achieved by using non-specific reagents as described in detail below (ionic strength, pH value, detergents). In addition, it can be tested whether a compound of interest can specifically elute the phosphorylated ZAP-70 from aminopyrido-pyrimidine ligand 24. Such ZAP-70 interacting compounds are described further in the following sections.

Such non-specific methods for destroying the interaction are principally known in the art and depend on the nature of the ligand enzyme interaction. Principally, change of ionic strength, the pH value, the temperature or incubation with detergents are suitable methods to dissociate the target enzymes from the immobilized ligand. The application of an elution buffer can dissociate binding partners by extremes of pH value (high or low pH; e.g. lowering pH by using 0.1 M citrate, pH2-3), change of ionic strength (e.g. high salt concentration using NaI, KI, MgCl2, or KCl), polarity reducing agents which disrupt hydrophobic interactions (e.g. dioxane or ethylene glycol), or denaturing agents (chaotropic salts or detergents such as Sodium-docedyl-sulfate, SDS; Review: Subramanian A., 2002, Immunoaffinty chromatography).

In some cases, the solid support has preferably to be separated from the released material. The individual methods for this depend on the nature of the solid support and are known in the art. If the support material is contained within a column the released material can be collected as column flowthrough. In case the support material is mixed with the lysate components (so called batch procedure) an additional separation step such as gentle centrifugation may be necessary and the released material is collected as supernatant. Alternatively magnetic beads can be used as solid support so that the beads can be eliminated from the sample by using a magnetic device.

In step d) of the method according to the first aspect of the invention, it is determined if phosphorylated ZAP-70 has been separated from the immobilized aminopyrido-pyrimidine ligand 24. This may include the detection of phosphorylated ZAP-70 or the determination of the amount of phosphorylated ZAP-70.

Consequently, at least in preferred embodiments of all identification methods of the invention, methods for the detection of separated phosphorylated ZAP-70 or for the determination of its amount are used. Such methods are known in the art and include physico-chemical methods such as protein sequencing (e.g. Edmann degradation), analysis by mass spectrometry methods or immunodetection methods employing antibodies directed against phosphorylated ZAP-70.

Preferably, phosphorylated ZAP-70 is detected or the amount of phosphorylated ZAP-70 is determined by mass spectrometry or immunodetection methods.

The identification of proteins with mass spectrometric analysis (mass spectrometry) is known in the art (Shevchenko et al., 1996, Analytical Chemistry 68: 850-858, (Mann et al., 2001, Analysis of proteins and proteomes by mass spectrometry, Annual Review of Biochemistry 70, 437-473) and is further illustrated in the example section.

Preferably, the mass spectrometry analysis is performed in a quantitative manner, for example by using iTRAQ technology (isobaric tags for relative and absolute quatification) or cICAT (cleavable isotope-coded affinity tags) (Wu et al., 2006. J. Proteome Res. 5, 651-658).

According to a further preferred embodiment of the present invention, the characterization by mass spectrometry (MS) is performed by the identification of proteotypic peptides of phosphorylated ZAP-70. The idea is that phosphorylated ZAP-70 is digested with proteases and the resulting peptides are determined by MS. As a result, peptide frequencies for peptides from the same source protein differ by a great degree, the most frequently observed peptides that "typically" contribute to the identification of this protein being termed "proteotypic peptide". Therefore, a proteotypic peptide as used in the present invention is an experimentally well observable peptide that uniquely identifies a specific protein or protein isoform.

According to a preferred embodiment, the characterization is performed by comparing the proteotypic peptides obtained in the course of practicing the methods of the invention with known proteotypic peptides. Since, when using fragments prepared by protease digestion for the identification of a protein in MS, usually the same proteotypic peptides are observed for a given enzyme, it is possible to compare the proteotypic peptides obtained for a given sample with the proteotypic peptides already known for enzymes of a given class of enzymes and thereby identifying the enzyme being present in the sample.

As an alternative to mass spectrometry analysis, the eluted phosphorylated ZAP-70 (including coeluted binding partners or scaffold proteins), can be detected or its amount can be determined by using a specific antibody directed against phosphorylated ZAP-70, preferably with an antibody recognizing a Tyrosine phosphorylation at position 493 of phosphorylated ZAP-70. Such antibody is known in the art (Cell Signaling Technologies, #2704).

Since aminopyrido-pyrimidine ligand 24 preferentially recognizes phosphorylated ZAP-70, It is also possible to use anti-ZAP-70 antibodies that recognize non-phosphorylated epitopes on ZAP-70.

Furthermore, in another preferred embodiment, once the identity of the coeluted binding partner has been established by mass spectrometry analysis, each binding partner can be detected with specific antibodies directed against this protein.

Suitable antibody-based assays include but are not limited to Western blots, ELISA assays, sandwich ELISA assays and antibody arrays or a combination thereof. The establishment of such assays is known in the art (Chapter 11, Immunology, pages 11-1 to 11-30 in: Short Protocols in Molecular Biology. Fourth Edition, Edited by F.M. Ausubel et al., Wiley, New York, 1999).

These assays can not only be configured in a way to detect and quantify a ZAP-70 interacting protein of interest (e.g. another kinase such as LAT which is a substrate of ZAP-70), but also to analyse posttranslational modification patterns such as ubiquitin modification.

Furthermore, the identification methods of the invention involve the use of compounds which are tested for their ability to be an ZAP-70 interacting compound.

Principally, according to the present invention, such a compound can be every molecule which is able to interact with ZAP-70, eg. by inhibiting its binding to aminopyrido-pyrimidine ligand 24. Preferably, the compound has an effect on ZAP-70, e.g. a stimulatory or inhibitory effect.

Preferably, said compound is selected from the group consisting of synthetic or naturally occurring chemical compounds or organic synthetic drugs, more preferably small molecules, organic drugs or natural small molecule compounds. Preferably, said compound is identified starting from a library containing such compounds. Then, in the course of the present invention, such a library is screened.

Such small molecules are preferably not proteins or nucleic acids. Preferably, small molecules exhibit a molecular weight of less than 1000 Da, more preferred less than 750 Da, most preferred less than 500 Da.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are provided in, for example, Breinbauer R, Manger M, Scheck M, Waldmann H. Natural product guided compound library development. Curr Med Chem. 2002 Dec;9(23):2129-45, wherein natural products are described that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fulfill the specific requirements of the individual binding pocket within a domain family it may be necessary to optimise the natural product structure by chemical variation. Solid-phase chemistry is said to become an efficient tool for this optimisation process, and recent advances in this field are highlighted in this review article. Other related references include Edwards PJ, Morrell AI. Solid-phase compound library synthesis in drug design and development. Curr Opin Drug Discov Devel. 2002 Jul;5(4):594-605.; Merlot C, Domine D, Church DJ. Fragment analysis in small molecule discovery. Curr Opin Drug Discov Devel. 2002 May;5(3):391-9. Review; Goodnow RA Jr. Current practices in generation of small molecule new leads. J Cell Biochem Suppl. 2001;Suppl 37:13-21; which describes that the current drug discovery processes in many pharmaceutical companies require large and growing collections of high quality lead structures for use in high throughput screening assays. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures.

In a preferred embodiment of the second aspect of the invention, phosphorylated ZAP-70 is first incubated with the compound and then with the immobilized aminopyrido-pyrimidine ligand 24.

Preferably, the phosphorylated ZAP-70 is first incubated with the compound for 10 to 60 minutes, more preferred 30 to 45 minutes at a temperature of 4°C to 37°C, more preferred 4°C to 25°C, most preferred 4°C. Preferably compounds are used at concentrations ranging from 1 µM to 1 mM, preferably from 10 to 100 µM. The second step, contacting with the immobilized ligand, is preferably performed for 10 to 60 minutes at 4°C.

Furthermore, steps a) to c) of the second aspect of the invention may be performed with several protein preparations in order to test different compounds. This embodiment is especially interesting in the context of medium or high troughput screenings (see below).

In a preferred embodiment of the method of the invention according to the third or forth aspect, a reduced amount of the aminopyrido-pyrimidine ligand 24-phosphorylated ZAP-70 complex formed in step c) in comparison to step b) indicates that phosphorylated ZAP-70 is a target of the compound. This results from the fact that in step c) of this method of the invention, the compound competes with the ligand for the binding of phosphorylated ZAP-70. If less phosphorylated ZAP-70 is present in the aliquot incubated with the compound, this means preferably that the compound has competed with the inhibitor for the interaction with the enzyme and is, therefore, a direct target of the enzyme and vice versa.

Preferably, the identification methods of the invention are performed as a medium or high throughput screening.

The interaction compound identified according to the present invention may be further charcterized by determining whether it has an effect on ZAP-70 activity, for example on its kinase activity (Isakov et al., The Journal of Biological Cheistry 271 (26), 15753-15761).

The compounds identified according to the present invention may further be optimized (lead optimisation). This subsequent optimisation of such compounds is often accelerated because of the structure-activity relationship (SAR) information encoded in these lead generation libraries. Lead optimisation is often facilitated due to the ready applicability of high-throughput chemistry (HTC) methods for follow-up synthesis.

One example of such a library and lead optimization is described in Wakeling AE, Barker AJ, Davies DH, Brown DS, Green LR, Cartlidge SA, Woodburn JR. Specific inhibition of epidermal growth factor receptor tyrosine kinase by 4-anilinoquinazolines. Breast Cancer Res Treat. 1996;38(1):67-73.

The specification further describes a method for the preparation of a pharmaceutical composition comprising the steps of
a) identifying a ZAP-70 interacting compound as described above, and
b) formulating the interacting compound to a pharmaceutical composition.

Therefore, the specification describes a method for the preparation of pharmaceutical compositions, which may be administered to a subject in an effective amount. The therapeutic may be substantially purified. The subject to be treated may be an animal including, but not limited to animals such as cows, pigs, horses, chickens, cats, dogs, etc., and may be a mammal, e.g. human or a non-human mammal.

The compounds identified according to the invention are useful for the prevention or treatment of diseases where ZAP-70 plays a role, e.g. immune or autoimmune diseases or disorders mediated by T lymphocytes. Such diseases or disorders comprise asthma, rheumatoid arthritis, psoriasis, inflammatory bowel disease (e.g. Crohn's disease or ulcerative colitis), multiple sclerosis, and acute or chronic rejection of organ or tissue allo- or xenografts.

In addition, the compounds of the invention can be used to treat or ZAP-70 positive B-cell chronic lymphocytic leukaemia (B-CLL).

In general, the pharmaceutical compositions of the present invention comprise a therapeutically effective amount of a therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc..

The amount of the therapeutic of the invention which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems. In general, suppositories may contain active ingredient in the range of 0.5% to 10% by weight; oral formulations preferably contain 10% to 95% active ingredient.

Various delivery systems are known and can be used to administer a therapeutic of the invention, e.g., encapsulation in liposomes, microparticles, and microcapsules: use of recombinant cells capable of expressing the therapeutic, use of receptor-mediated endocytosis (e.g., Wu and Wu, 1987, J. Biol. Chem. 262:4429-4432); construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents: Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions of the invention locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the therapeutic can be delivered in a vesicle, in particular a liposome (Langer, 1990, Science 249:1527-1533).

In yet another embodiment, the therapeutic can be delivered via a controlled release system. In one embodiment, a pump may be used (Langer, supra). In yet another embodiment, a controlled release system can be placed in proximity of the therapeutic target, i.e., the brain, thus requiring only a fraction of the systemic dose

The invention further relates to a method for the purification of phosphorylated ZAP-70, comprising the steps of
a) providing a protein preparation containing phosphorylated ZAP-70,
b) contacting the protein preparation with aminopyrido-pyrimidine ligand 24 immobilized on a solid support under conditions allowing the formation of an aminopyrido-pyrimidine ligand 24-phosphorylated ZAP-70 complex, and
c) separating phosphorylated ZAP-70 from the immobilized aminopyrido-pyrimidine ligand 24.

As mentioned above, it has been surprisingly found that aminopyrido-pyrimidine ligand 24 is a ligand which recognizes phosphorylated ZAP-70. This enables efficient purification methods for phosphorylated ZAP-70.

With respect to phosphorylated ZAP-70, the protein peparation containing phosphorylated ZAP-70, the conditions for contacting with aminopyrido-pyrimidine ligand 24, immobilized aminopyrido-pyrimidine ligand 24, the aminopyrido-pyrimidine ligand 24-phosphorylated ZAP-70 complex, the separation of phosphorylated ZAP-70 from the immobilized aminopyrido-pyrimidine ligand 24, and the detection of phosphorylated ZAP-70 or the determination of its amount, the embodiments as defined above for the identification methods of the invention also apply to the purification method of the invention.

In a preferred embodiment, the purification method of the invention further comprises after step c) the identification of proteins being capable of binding to phosphorylated ZAP-70. This is especially interesting when the formation of the complex is performed under essentially physiological conditions, because it is then possible to preserve the natural condition of the enzyme which includes the existence of binding partners, enzyme subunits or post-translational modifications, which can then be identified with the help of mass spectrometry (MS).

Consequently, in a preferred embodiment, the purification method of the invention further comprises after step c) the determination whether the phosphorylated ZAP-70 is further posttranslationally modified, e.g. by ubiquitine modification.

The invention further relates to the use of aminopyrido-pyrimidine ligand 24 for the identification of ZAP-70 interacting compounds and for the purification of phosphorylated ZAP-70. The embodiments as defined above also apply to the uses of the invention.

It has been described that ZAP-70 is a prognostic marker for chronic lymphocytic leukaemia (CLL) which represents a B-cell malignancy (Hamblin and Hamblin, 2005, Expert Opinion in Therapeutic Targets 9(6):1165-1178). Originally CLL subtypes were distinguished by the mutational status of the immunoglobulin variable region genes, but it is now established that a CLL subtype can be identified by the expression of ZAP-70. The ZAP-70 kinase is normally expressed in T cells rather than B cells, but it is anomalously expressed in the more aggressive subtype of CLL Consequently, the aminopyrido-pyrimidine ligand 24 is an important tool for the diagnosis of subtypes of CLL or for the prognosis of disease progression by determining the probability that a CLL patient will suffer from advanced stage disease.

Therefore, in a further aspect, the specification also describes the use of the aminopyrido-pyrimidine ligand 24 for the preparation of a diagnosticum for chronic lymphocytic leukaemia (CLL).

The specification further describes a method for the diagnosis/prognosis of CLL, comprising the step of determining the presence/amount of phosphorylated ZAP-70 in a sample in relation to the total amount of ZAP-70, wherein an increased amount of phosphorylated ZAP-70 in comparison to the total amount of ZAP-70 (non-phosphorylated plus phosphorylated ZAP-70) in a sample from a CLL patient is indicative for having a higher probability for an aggressive form of CLL.

In this context, a sample is a protein preparation derived from the subject. Consequently, all embodiments discussed above with respect to protein preparation derive from subjects also apply to this aspect of the invention.

According to the invention, "subject" means any mammal, preferably a human being.

The amount of phosphorylated ZAP-70 can be determined as described above.

In the context of the present invention, "diagnosis" means both the diagnosis of an existing disease as well as the prognosis that a given subject has a probability of more than 50 % to develop the respective disease.

The invention is further illustrated by the following figures and examples, which are not considered as being limiting for the scope of protection conferred by the claims of the present application.

### Short description of the figures

**Figure 1****:** Structure of aminopyrido-pyrimidin ligand 24.
   The free primary amino group can be used for covalent coupling to a solid support material.
**Figure 2****:** Drug pulldown experiment with immobilized aminopyrido-pyrimidin ligand 24 and Western blot analysis.
   As biological material cell lysates prepared from non-treated or pervanadate treated Jurkat cells were used. The drug pulldown experiment was performed as described in Example 2 with lysate samples containing 50 mg of protein. Input lysate, flowthrough (non-bound fraction) and SDS-eluates (bound fraction) were separated on a SDS-polyacrylamide gel and transferred to a membrane. The blot was probed first with a general anti-ZAP-70 antibody (2A) and then with an antiphospho-ZAP-70 antibody (2B). Secondary detection antibodies were labeled with fluorescent dyes for detection with the Odyssey infrared imaging system.
   Lysates from non-treated Jurkat cells (lanes 1 to 3):
   Lane 1: Lysate control, not subjected to drug pulldown experiment
   Lane 2: Flow-through (non-bound fraction)
   Lane 3: Eluate (SDS-eluted bound fraction)
   Lysates from pervanadate-treated Jurkat cells (lanes 4 to 6):
   Lane 4: Lysate control, not subjected to drug pulldown experiment
   Lane 5: Flow-through (non-bound fraction)
   Lane 6: Eluate (SDS-eluted bound fraction)
   2A: The upper blot was probed with the general ZAP-70 antibody (L1E5, mouse monoclonal antibody directed against residues surrounding the aminoterminal part of human ZAP-70, Cell Signaling #2709).
   2B: The lower blot was probed with the phospho-ZAP-70 antibody (Phospho-ZAP-70 Tyr493, Cell Signaling #2704).

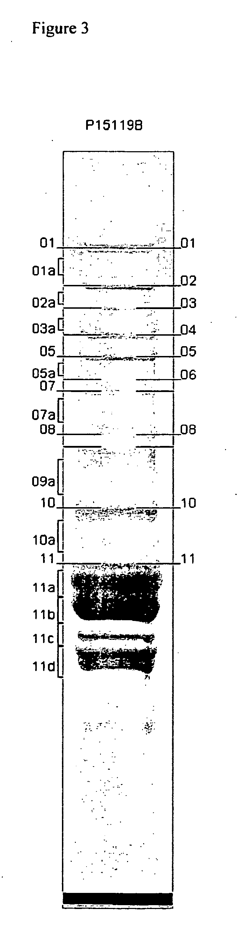

**Figure 3****:** Drug pulldown experiment with immobilized aminopyrido-pyrimidin ligand 24 for mass spectrometry analysis of proteins and phosphopeptides.
   Proteins bound to immobilized aminopyrido-pyrimidin ligand 24 were eluted with SDS sample buffer and analyzed by SDS-polyacrylamide gel electrophoresis (SDS-PAGE). A protein gel after staining with Coomassie blue is shown. The indicated gel areas were cut out as gel slices and proteins were subjected to analysis by mass spectrometry. The position of ZAP-70 is in gel slice 10 and 10a.
**Figure 4****:** Peptides identified of ZAP-70.
   The peptides that were identified by mass spectrometry analysis of the human ZAP-70 sequence (IPI00329789.5, 628 amino acids) are shown in bold type and underlined.

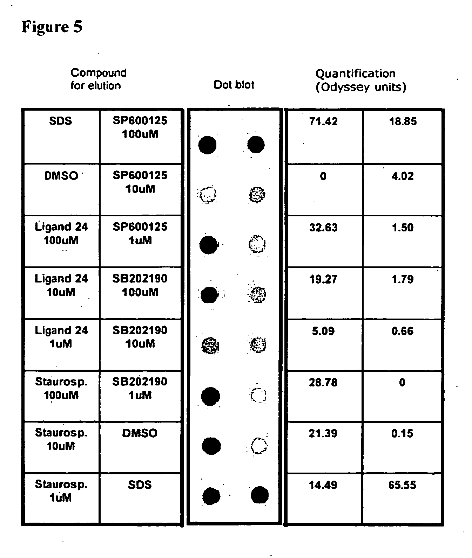

**Figure 5****:** Assay for the identification of ZAP-70 interacting compounds.
   The experiment was performed as described in example 3. ZAP-70 protein was captured by immobilized aminopyrido-pyrimidin ligand 24 from Jurkat cell lysates (pervanadate treated Jurkat cells) and eluted by the compounds as indicated. Eluates were transferred to a nitrocellulose membrane and ZAP-70 was detected with the Odyssey Infrared Imaging system. First antibody: anti-phosphoZAP-70 (Tyr493). Second antibody: goat anti-rabbit Irdye800CW. Relative Odyssey units are shown. Compounds used for elution: SDS, positive control for maximal elution; DMSO, solvent control; ligand 24, aminopyrido-pyrimidin ligand 24; Staurosporine; JNK inhibitor SP600125; p38 inhibitor SB 202190.

### Example 1: Preparation of affinity matrix

This example illustrates the preparation of an affinity matrix for affinity capture of kinases from cell lysates. A capturing ligand was covalently immobilized on a solid support through covalent linkage using an amino functional group. This affinity matrix was used in example 2 and example 3.

### Synthesis of aminopyrido-pyrimidin ligand 24: 7-(4-Aminomethyl-phenylamino)-3-(2,6-dichloro-phenyl)-1-methyl-1H-[1,6]naphthyridin-2-one

The first seven steps of the synthesis were performed as described in Klutchko, S.R. et al., 1998, Journal of Medicinal Chemistry 41, 3276-3292. The remaining steps were performed as described below.

**Steps 1-7:** 6-(2,6-Dichlorophenyl)-2-methanesulfonyl-8-methyl-8*H*-pyrido[2,3-*d*]pyrimidin-7-one was synthesized from 4-chloro-2-methylsulfanyl-5-pyrimidinecarboxylate ethyl ester following the procedure in J. Med. Chem. 1998, 41, 3276-3292.

### Step 8: {4-[3-(2,6-Dichloro-phenyl)-1-methyl-2-oxo-1,2-dihydro-[1,6]naphthyridin-7 ylamino] benzyl}-carbamic acid tert-butyl ester

6-(2,6-Dichlorophenyl)-2-methanesulfonyl-8-methyl-8*H*-pyrido[2,3-*d*]pyrimidin-7-one (0.100g, 0.2mmol) and 3-(*N*-Boc-methylamino)aniline (0.421g, 2.0mmol) were mixed as solids and heated to 140°C for 30 mins. The crude reaction mixture was dissolved in dichloromethane and washed with 2N HCl (aq) x 2. The organic layer was dried with anhydrous magnesium sulfate, filtered and concentrated. The crude product was recrystallised from hot ethyl acetate to afford {4-[3-(2,6-Dichloro-phenyl)-1-methyl-2-oxo-1,2-dihydro-[1,6]naphthyridin-7-ylamino] benzyl}-carbamic acid *tert*-butyl ester as a yellow solid (0.031g- 25%). ¹H NMR (DMSO-*d*₆) δ 10.18 (s, 1H); 8.83 (s, 1H); 7.76 (d, 2H); 7.58 (d, 2H); 7.46 (dd, 1H); 7.32 (brt, 1H); 7.23 (d, 2H); 4.10 (d, 2H); 3.66 (s, 3H); 1.40 (s, 9H). LCMS: method A, RT=5.60min.

### Step 9: 7-(4-Aminomethyl-phenylamino)-3-(2,6-dichloro-phenyl)-1-methyl-1H-[1,6]naphthyridin-2-one

{4-[3-(2,6-Dichloro-phenyl)-1-methyl-2-oxo-1,2-dihydro-[1,6]naphthyridin-7-ylamino] benzyl}-carbamic acid *tert*-butyl ester (0.026g, 0.05mmol) was dissolved in methanol

(3ml) and hydrochloric acid (4N in dioxane, 1.2ml) was added. The reaction was stirred at room temperature for 1.5 hours when HPLC showed no remaining starting material. The solvent was removed *in vacuo*. The residue was dissolved in water and the solution basified with sodium carbonate (sat., aq.). The resulting precipitate was collected and dried to afford 7-(4-Aminomethyl-phenylamino)-3-(2,6-dichloro-phenyl)-1-methyl-1H-[1,6]naphthyridin-2-one (0.021g -100%) as a yellow solid. ¹H NMR (DMSO-*d*₆) δ 10.20 (bid, 1H); 8.83 (d, 1H); 7.90 (d, 1H); 7.76 (d, 1H); 7.72 (d, 1H); 7.60 (dd, 2H); 7.47 (ddd, 1H); 7.33 (d, 1H); 7.24 (d, 1H); 4.07 (d, 2H); 3.66 (s, 3H). LCMS: method A, RT=4.44min, [MH⁺=426].

All reactions were carried out under inert atmosphere. NMR spectra were obtained on a Bruker dpx400. LCMS was carried out on an Agilent 1100 using a zorbax SBC-18, 4.6mmx150mm-5µ column. Column flow was 1ml/min and solvents used were water and acetonitrile (0.1%TFA) with an injection volume of 10ul. Wavelengths were 254 and 210nm. Methods are described below.

**Table 1: Analytical methods**

| **Method** | **Easy Access** | **ChemStation** | **Flow** | **Solvent** | **Run** |
|---|---|---|---|---|---|
| | **Method Name** | **Method Name** | **Rate** | | **Time** |
| A | Analytical positive | ANL_POS7.M | 1ml/min | 0-2.5min | 7 min |
| | 7mn | | | 5-95% MeCN | |
| | | | | | |
| | | | | 2.5-6min | |
| | | | | 95% MeCN | |
| B | Analytical positive | ANAL_POS.M | 1ml/min | 0-11min | 15 min |
| | Ion | | | 5-95% MeCN | |
| | | | | | |
| | | | | 11-13min | |
| | | | | 95% MeCN | |

**Table 2: Abbreviations used in chemistry protocols**

| | |
|---|---|
| aq | aqueous |
| d | doublet |
| DMSO | dimethyl sulfoxide |
| g | gram |
| HCl | Hydrochloric acid |
| HPLC | high pressure liquid chromatography |
| LCMS | liquid chromatography - mass spectrometry |
| m | multiplet |
| mins | minute |
| mmol | millimole |
| N | Normal |
| NMR | nuclear magnetic resonance |
| q | quartet |
| RT | retention time |
| s | singlet |
| sat | saturated |
| t | triplet |

### Immobilization of ligand containing amine group

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads was placed in a 15 ml Falcon tube, compound stock solution (usually 100 mM in DMF or DMSO) was added (final concentration 0.2-2 µmol/ml beads) as well as 15 µl of triethylamine (Sigma, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at room temperature on the end-over-end shaker over night. Beads were washed with 10 ml of DMSO and were stored in isopropanol at -20°C. These beads were used as the affinity matrix in example 2 and 3. Control beads (no ligand immobilized) were generated by blocking the NHS-groups by incubation with aminoethanol as described above.

### Example 2: Drug pulldown of a phosphorylated form of ZAP-70 using immobilized aminopyrido-pyrimidin ligand 24

This example demonstrates the use of the immobilized aminopyrido-pyrimidin ligand 24 for the identification of ZAP-70 from cell lysates of a human T cell line (Jurkat cells). To this end lysate of non-treated and treated Jukat cells was contacted with the affinity matrix described in example 1. Proteins binding to the aminopyrido-pyrimidin ligand 24 were identified by Western blot and mass spectrometry (MS) analysis.

For Western blot analysis bound proteins were eluted from the affinity matrix and subsequently separated by SDS-Polyacrylamide gel elecrophoresis. ZAP-70 was detected with a general monoclonal antibody binding to the non-phosphorylated aminoterminal part of the protein and in addition with a phospho-specific antibody (Figure 2).

The results of the Western blot analysis show that immobilized aminopyrido-pyrimidin ligand 24 preferentially pulls down a phosphorylated form of ZAP-70. The phosphorylated ZAP-70 was only significantly detected from treated Jurkat cell lysates compared to non-treated lysates.

For the identification of proteins and phosphopeptides by mass spectrometry analysis proteins captured by the affinty matrix were eluted and subsequently separated by SDS-Polyacrylamide gel elecrophoresis. Suitable gel bands were cut out and subjected to in-gel proteolytic digestion with trypsin. Phosphorylated peptides where then enriched via immobilized metal affinity chromatography (IMAC). Retained phospho-peptides and non-bound peptides were separately analyzed by LC-MS/MS mass spectrometry.

The peptide sequence coverage of ZAP-70 is shown in Figure 4. In total 9 ZAP-70 phosphopeptides were identified by mass spectrometry (Table 4).

### 1. Cell culture

Jurkat cells (clone E6-1 from ATCC, number TIB-152) were grown in 1 litre Spinner flasks (Integra Biosciences, #182101) in suspension in RPMI 1640 medium (Invitrogen, #21875-034) supplemented with 10% Fetal Bovine Serum (Invitrogen) at a density between 0.15 x 10e6 and 1.2 x 10e6 cells/ml. For stimulation experiments cells were treated with 30 µM pervanadate (final concentration) at a density of about 1.0 x 10e6 cells/ml for one hour and subsequently harvested by centrifugation. After intensive washing with 1 x PBS buffer (Invitrogen, #14190-094) cell pellets were frozen in liquid nitrogen and subsequently stored at -80°C.

For stimulation experiments cells were treated with H₂O₂ treated sodium orthovanadate in order to inhibit protein tyrosine phosphatases (D.C. Weiser and S. Shenolikar, Chapter 18.10 in Current Protocols in Molecular Biology, 2003, John Wiley & Sons, Inc.). For cell treatment 30 ml of mix 3 was added to 10 liters of culture medium resulting in a final concentration of 30 µM pervanadate. Jurkat cells were kept at a density between 0.8 to 1.2 x 10e6 cells/ml for one hour and then harvested.

### Preparation of pervanadate solutions

Dissolve sodium orthovanadate (Sigma, #6508) at 100 mM final concentration in sterile, distilled water and adjust the pH value of the solution to pH 10.0 with 0.1 M NaOH. Place the solution in a boiling water bath until the solution clarifies or becomes translucent. If necessary, readjust pH to 10.0 and store the stock solution at -20°C. Avoid repeated rounds of freezing and thawing of the stock solution.
Mix 500 µl of 30% H₂O₂ (Sigma, Cat. No. H1009) and 375 µl 1 x PBS (Mix 1).
Mix 3.5 ml 100 mM vanadate solution (pH 10.0) with 31.5 ml 1 x PBS (Mix 2).
Mix 650 µl Mix 1 and 32.5 ml Mix 2 (Mix 3). Incubate Mix 3 for 10 minutes at room temperature before applying to cell cultures.

### 2. Preparation of cell lysates

Jurkat cells were homogenized in a Potter S homogenizer in lysis buffer: 50 mM Tris-HCl, 0.8% NP40, 5% glycerol, 150 mM NaCl, 1.5 mM MgCl2, 25 mM NaF, 1 mM sodium vanadate, 1 mM DTT, pH 7.5. One complete EDTA-free tablet (protease inhibitor cocktail, Roche Diagnostics, 1 873 580) per 25 ml buffer was added. The material was dounced 10 times using a mechanized POTTER S, transferred to 50 ml falcon tubes, incubated for 30 minutes on ice and spun down for 10 min at 20,000 g at 4°C (10,000 rpm in Sorvall SLA600, precooled). The supernatant was transferred to an ultracentrifuge (UZ)-polycarbonate tube (Beckmann, 355654) and spun for 1 hour at 100.000 g at 4°C (33.500 rpm in Ti50.2, precooled). The supernatant was transferred again to a fresh 50 ml falcon tube, the protein concentration was determined by a Bradford assay (BioRad) and samples containing 50 mg of protein per aliquot were prepared. The samples were immediately used for experiments or frozen in liquid nitrogen and stored frozen at -80°C.

### 3. Compound pull-down experiment

Sepharose-beads with immobilized compound (100 µl beads per pull-down experiment) were equilibrated in lysis buffer and incubated with a cell lysate sample containing 50 mg of protein on an end-over-end shaker (Roto Shake Genie, Scientific Industries Inc.) for 2 hours at 4°C. Beads were collected, transfered to Mobicol-columns (MoBiTech 10055) and washed with 10 ml lysis buffer containing 0.5% NP40 detergent, followed by 5 ml lysis buffer with 0.25 % detergent. To elute the bound protein, 60 µl 2 x SDS sample buffer was added, the column was heated for 30 minutes at 50°C and the eluate was transferred to a microfuge tube by centrifugation. Proteins were then separated by SDS-Polyacrylamide electrophoresis (SDS-PAGE).

### 4. Protein detection by Western blot analysis

Western blots were performed according to standard procedures and the ZAP-70 protein was detected and quantified by using a specific anti-phospho ZAP-70 antibody (first antibody), a fluorescently labeled secondary antibody and the Odyssey Infrared Imaging system from LI-COR Biosciences (Lincoln, Nebraska, USA) according to instructions provided by the manufacturer (Schutz-Geschwendener et al., 2004. Quantitative, two-color Western blot detection with infrared fluorescence. Published May 2004 by LI-COR Biosciences, www.licor.com).

The phospho-ZAP-70 antibody (pTyr493, rabbit polyclonal, Cell Signaling #2704) detects ZAP-70 only when phosphorylated at tyrosine residue 493. The general ZAP-70 antibody (L1E5, mouse monoclonal antibody, Cell Signaling #2709) is directed against the amino-terminus of human ZAP-70.

### 5. Protein Identification by Mass Spectrometry

### 5.1 Protein digestion prior to mass spectrometric analysis

Gel-separated proteins were reduced, alkylated and digested in gel essentially following the procedure described by Shevchenko et al., 1996, Anal. Chem. 68:850-858. Briefly, gel-separated proteins were excised from the gel using a clean scalpel, reduced using 10 mM DTT (in 5mM ammonium bicarbonate, 54°C, 45 min) and subsequently alkylated with 55 mM iodoacetamid (in 5 mM ammonium bicarbonate) at room temperature in the dark (30 minutes). Reduced and alkylated proteins were digested in gel with porcine trypsin (Promega) at a protease concentration of 12.5 ng/µl in 5mM ammonium bicarbonate. Digestion was allowed to proceed for 4 hours at 37°C and the reaction was subsequently stopped using 5 µl 5% formic acid.

### 5.2 Sample preparation prior to analysis by mass spectrometry

Gel plugs were extracted twice with 20 µl 1% TFA and pooled with acidified digest supernatants. Samples were dried in a a vaccuum centrifuge and resuspended in 10 µl 0.1% formic acid. Dried samples were resuspended in 30 µl of a water containing 250 mM acetic acid, 30% acetonitrile and 4 µl PHOS-select Iron Affinity beads (Sigma, P9740; IMAC material) and incubated for 2 hours at room temperature under gentle horizontal shaking. Subsequently, the slurry was loaded onto a gel loader tip containing a restriction to hold back the IMAC material. Beads were allowed to settle for a minute, then the solvent was pushed through the column using a P10 pipette and collected in an Eppendorf tube. The IMAC column was washed two times with 20 µl of an aqueous solution containing 250 mM acetic acid, 30% acetonitrile. The eluted fractions were combined and dried in a vaccum centrifuge. This fraction is referred to as the non-bound fraction (NBF). Phosphopeptides were eluted from the IMAC column using 20 µl of an aqueous solution containing 400 mM ammonia and 30% acetonitrile. The elution was repeated twice and the eluted fractions were combined and dried in a vacuum centrifuge (bound fraction, BF). Both fractions (NBF and BF) were resuspended in 10 µl 0.1% formic acid prior to LC-MS/MS analysis (Pozuelo Rubio et al., 2005, Biochemical Journal 392(Part 1), 163-172).

### 5.3. Mass spectrometric data acquisition

Peptide samples were injected into a nano LC system (CapLC, Waters or Ultimate, Dionex) which was directly coupled either to a quadrupole TOF (QTOF2, QTOF Ultima, QTOF Micro, Micromass) or ion trap (LCQ Deca XP) mass spectrometer. Peptides were separated on the LC system using a gradient of aqueous and organic solvents (see below).

Solvent A was 5% acetonitrile in 0.5% formic acid and solvent B was 70% acetonitrile in 0.5% formic acid.

**Table 3: Peptides eluting off the LC system were partially sequenced within the mass spectrometer.**

| Time (min) | % solvent A | % solvent B |
|---|---|---|
| 0 | 95 | 5 |
| 5.33 | 92 | 8 |
| 35 | 50 | 50 |
| 36 | 20 | 80 |
| 40 | 20 | 80 |
| 41 | 95 | 5 |
| 50 | 95 | 5 |

### 5.4. Protein identification

The peptide mass and fragmentation data generated in the LC-MS/MS experiments were used to query fasta formatted protein and nucleotide sequence databases maintained and updated regularly at the NCBI (for the NCBInr, dbEST and the human and mouse genomes) and European Bioinformatics Institute (EBI, for the human, mouse, D. melanogaster and C. elegans proteome databases). Proteins were identified by correlating the measured peptide mass and fragmentation data with the same data computed from the entries in the database using the software tool Mascot (Matrix Science; Perkins et al., 1999. Probability-based protein identification by searching sequence databases using mass spectrometry data. Electrophoresis 20, 3551-3567). Search criteria varied depending on which mass spectrometer was used for the analysis.

### 5.5 Phosphopeptide identification

The identification of phosphorylated peptides was achieved by allowing phosphorylation at Serine, Threonine and Tyrosine.residues as variable modifications in Mascot search parameters followed by manual inspection of the spectrum to sequence assignments.

**Table 4: Phospho-peptides of ZAP-70**

| The phospho-peptides that were identified by mass spectrometry analysis of the human | | | | | | | |
|---|---|---|---|---|---|---|---|
| ZAP protein sequence (IPI00329789.5, 628 amino acids) are indicated (Observed value: | | | | | | | |
| measured mass to charge ratio; Mr(exp.): 'observed value' x charge). | | | | | | | |
| Peptide number | Start position | End position | Observed value | Mr(expt) | Mr(calc) | Delta | Sequence |
| 1 | 176 | 186 | 624.297 | 1246.579 | 1246.559 | 0.02 | KLpYSGAQTDGK |
| 2 | 241 | 251 | 687.349 | 1372.684 | 1372.683 | 0.002 | LKADGLIpYCLK |
| 3 | 283 | 298 | 628.966 | 1883.876 | 1883.841 | 0.034 | RIDTLNSDGpYTPEPAR |
| 4 | 284 | 298 | 864.899 | 1727.783 | 1727.74 | 0.043 | IDTLNSDGpYTPEPAR |
| 5 | 299 | 306 | 497.244 | 992.474 | 992.469 | 0.005 | ITpSPDKPR |
| 6 | 485 | 496 | 691.795 | 1381.575 | 1381.555 | 0.02 | ALGADDSpYYTAR |
| 7 | 485 | 496 | 691.8 | 1381.585 | 1381.555 | 0.03 | ALGADDSYpYTAR |
| 8 | 485 | 496 | 731.792 | 1461.569 | 1461.521 | 0.048 | ALGADDSpYpYTAR |
| 9 | 604 | 612 | 571.749 | 1141.483 | 1141.451 | 0.032 | ACYpYSLASK |

### Example 3: Assay for the identification of ZAP-70 interacting compounds

The preparation of the aminopyrido-pyrimidin ligand 24 affinity matrix was done as described in example 1. To screen maximally 80 compounds in a 96 well plate the elution experiment is performed as described below.

### Elution assay

The affinity matrix (1600 µl of beads) was washed 2 x with 30 ml 1x DP-buffer. After each washing step the beads were collected by centrifugation for 4 minutes at 1300 rpm at 4°C in a Heraeus centrifuge. The supernatants were discarded. Finally, the beads were equilibrated in 50 ml binding buffer (1x DP buffer/0.4% NP40), and rotated for 30 to 45 minutes on a ROTO SHAKE GENIE (Scientific Industries, Inc.) at 4°C. After this incubation time the beads were harvested and mixed with 45 ml of Pervanadate activated cleared Jurkat cell lysate at a protein concentration of 5 mg/ml. The preparation of the lysate was done as described in example 2. Beads and the lysate were incubated for 2 hours at 4°C. After the incubation with the lysate beads were collected by centrifugation as described and washed three times with 25 ml binding buffer. During each washing step the beads were incubated for 8 minutes on a ROTO SHAKE GENIE (Scientific Industries, Inc.) at 4°C. After the third wash the beads were transferred to 2 ml columns (MoBiTec, #S10129) and washed with 20 ml 1 x DP buffer/0.2% NP40. Once the washing buffer had run through the column completely the volume of beads left in the column was calculated (approximately 1000 µl). The beads were resuspended in 4 fold excess of 1x DP-buffer/0.2% NP40 (4 ml) to generate a 1:4 slurry. For compound elution tests 50 µl of this suspension was added to each well of a 96 well plate (Millipore MultiScreenHTS, MSBVN1210, with lid and 1.2um hydrophilic low protein binding Durapore membrane). To remove residual buffer the 96 well plate was assembled with Assemble filter and collection plate and this sandwich assembly was spun down for 10 seconds at 800 rpm in a centrifuge. Then 40 µl of elution buffer (1x DP-buffer/0.2% NP40) supplemented with the test compound was added to the beads. Test compounds were prepared by diluting them in dilution buffer starting from 40 fold concentrated stock solution in DMSO. The plate was assembled on the collection plate, fixed on an Eppendorf incubator and incubated for 30 minutes at 4°C at 650 rpm shaking. To harvest the eluate the 96 well filter plate assembled on the 96 well collection plate was centrifuged for 1 minute at 800 rpm in a table top centrifuge at 4°C (Heraeus). The typical volume of the eluate is approximately 35 µl. Eluates were stored at -80°C. The eluates were checked for the presence of ZAP-70 by using a dot blot procedure.

As test compounds, Aminopyrido-pyrimidin ligand 24 (see Example 1), Staurosporine (Sigma S4400), JNK inhibitor SP600125 (Calbiochem 420119) and p38 inhibitor SB 202190 (Tocris 1264) were used. Typically all compounds are dissolved in 100 % DMSO (Fluka, cat.no 41647) at a concentration of 100 mM. Alternatively, 100 % DMF (Fluka, cat.no 40228) can be used for those compounds which cannot be dissolved in DMSO. Compounds are stored at -20°C. Dilution of test compound for elution experiments: Prepare 50 mM stock by diluting the 100 mM stock 1:1 with 100% DMSO. For elution experiments further dilute the compound with elution buffer (1x DP-buffer, 0.2% NP40).

### Preparation of buffers

**Table 5: Preparation of 5x-DP buffer**

| **Substance:** | **Stock solution** | **Final conc. in 1 x** | **Add for 1l 5 x lysis** |
|---|---|---|---|
| | | **lysis buffer** | **buffer** |
| **Tris/HCl pH 7.5** | 1 M | 50 mM | 250 ml |
| **Glycerol** | 87 % | 5 % | 288 ml |
| **MgCl₂** | 1M | 1.5 mM | 7.5 ml |
| **NaCl** | 5 M | 150 mM | 150 ml |
| **Na₃VO₄** | 100 mM | 1 mM | 50 ml |

The 5x-DP buffer was filtered through 0.22 µm filter and stored in 40 ml-aliquots at -80°C. These solutions were obtained from the following suppliers: 1.0 M Tris/HCl pH 7.5 (Sigma, T-2663), 87% Glycerol (Merck, catalogue number 04091.2500); 1.0 M MgCl₂ (Sigma, M-1028); 5.0 M NaCl (Sigma, S-5150).

### Detection of eluted ZAP-70

The eluted ZAP-70 protein was detected and quantified by a dot blot procedure using a specific anti-phospho ZAP-70 antibody (first antibody), a fluorescently labeled secondary antibody and the Odyssey Infrared Imaging system from LI-COR Biosciences (Lincoln, Nebraska, USA) according to instructions provided by the manufacturer (Schutz-Geschwendener et al., 2004. Quantitative, two-color Western blot detection with infrared fluorescence. Publishe May 2004 by LI-COR Biosciences, www.licor.com).

Nitrocellulose membranes were treated with 20% ethanol for 5 seconds and subsequently washed with 1 x PBS buffer. Eluates (as described above) were combined with 10 µl of 4 x SDS loading buffer (200 mM Tris-HCl pH6.8, 8% SDS, 40% glycerol, 0.04% Bromphenol blue) and applied to the Nitrocellulose membrane with a BioRad dot blot appartus (BioRad, #170-6545) and washed once with 1 x PBS buffer.

For detection of ZAP-70 protein the membranes were first blocked by incubation with Odyssey blocking buffer for 1 hour. Blocked membranes were incubated for 16 hours at 4°C with the first antibody, a rabbit anti-phopspho ZAP-70 antibody recognizing phosphorylated Tyrosine residue 493 (pTyr493, rabbit polyclonal, Cell Signaling #2704) diluted 1:1000 in Odyssey blocking buffer supplemented with 0.1% Tween 20 and 0.02% SDS.

After washing the membrane four times for 5 minutes with 1 x PBS buffer containing 0.01% Tween 20 the membrane was incubated for 1 hour with the detection antibody (Goat anti-rabbit IRDye 800CW antibody from LI-COR, diluted 1:10 000 in Odyssey Blocking Buffer supplemented with 0.1% Tween 20 and 0.02% SDS). Afterwards the membrane was washed four times for 5 minutes with 1 x PBS buffer/0.1% Tween 20 and once for 5 minutes with 1 x PBS buffer. Afterwards the membrane was scanned with the Odyssey reader and data were analysed.

## Claims

1. A method for the identification of an ZAP-70 interacting compound, comprising the steps of
a) providing a protein preparation containing phosphorylated ZAP-70,
b) contacting the protein preparation with 7-(4-Aminomethyl-phenylamino)-3-(2,6-dichloro-phenyl)-1-methyl-1H-[1,6]naphthyridin-2-one immobilized on a solid support under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex,
c) incubating the 7-(4-Aminomethyl-phenylamino)-3-(2,6-dichloro-phenyl)-1-methyl-1H-[1,6]naphthyridin-2-one - phosphorylated ZAP-70 complex with a given compound, and
d) determining whether the compound is able to separate phosphorylated ZAP-70 from the immobilized 7-(4-Aminomethyl-phenylamino)-3-(2,6-dichlorophenyl)-1-methyl-1H-[1,6]naphthyridin-2-one.

2. The method of claim 1, wherein step d) includes the detection of separated phosphorylated ZAP-70 or the determination of the amount of separated phosphorylated ZAP-70.

3. The method of claim 2, wherein separated phosphorylated ZAP-70 is detected or the amount of separated phosphorylated ZAP-70 is determined by mass spectrometry or immunodetection methods.

4. The method of claim 3, wherein separated phosphorylated ZAP-70 is detected with an antibody directed against phosphorylated ZAP-70.

5. The method of claim 4, wherein separated phosphorylated ZAP-70 is detected with an antibody recognizing a phosphorylation at position 493 of phosphorylated ZAP-70.

6. A method for the identification of an ZAP-70 interacting compound, comprising the steps of
a) providing a protein preparation containing phosphorylated ZAP-70,
b) contacting the protein preparation with 7-(4-Aminomethyl-phenylamino)-3-(2,6-dichloro-phenyl)-1-methyl-1H-[1,6]naphthyridin-2-one immobilized on a solid support and with a given compound under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex, and
c) detecting the 7-(4-Aminomethyl-phenylamino)-3-(2,6-dichloro-phenyl)-1-methyl-1H-[1,6]naphthyridin-2-one - phosphorylated ZAP-70 complex formed in step b).

7. The method of claim 6, wherein in step c) said detecting is performed by determining the amount of the 7-(4-Aminomethyl-phenylamino)-3-(2,6-dichlorophenyl)-1-methyl-1H-[1,6]naphthyridin-2-one - phosphorylated ZAP-70 complex.

8. The method of any of claims 6 or 7, wherein steps a) to c) are performed with several protein preparations in order to test different compounds.

9. A method for the identification of a ZAP-70 interacting compound, comprising the steps of:
a) providing two aliquots of a protein preparation containing phosphorylated ZAP-70,
b) contacting one aliquot with 7-(4-Aminomethyl-phenylamino)-3-(2,6-dichlorophenyl)-1-methyl-1H-[1,6]naphthyridin-2-one immobilized on a solid support under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex,
c) contacting the other aliquot with 7-(4-Aminomethyl-phenylamino)-3-(2,6-dichloro-phenyl)-1-methyl-1H-[1,6]naphthyridin-2-one immobilized on a solid support and with a given compound under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex, and
d) determining the amount of 7-(4-Aminomethyl-phenylamino)-3-(2,6-dichlorophenyl)-1-methyl-1H-[1,6]naphthyridin-2-one- phosphorylated ZAP-70 complex formed in steps b) and c).

10. A method for the identification of a ZAP-70 interacting compound, comprising the steps of:
a) providing two aliquots comprising each at least one cell containing phosphorylated ZAP-70,
b) incubating one aliquot with a given compound,
c) harvesting the cells of each aliquot,
d) lysing the cells in order to obtain protein preparations,
e) contacting the protein preparations with 7-(4-Aminomethyl-phenylamino)-3-(2,6-dichloro-phenyl)-1-methyl-1H-[1,6]naphthyridin-2-one immobilized on a solid support under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex, and
f) determining the amount of 7-(4-Aminomethyl-phenylamino)-3-(2,6-dichlorophenyl)-1-methyl-1H-[1,6]naphthyridin-2-one-phosphorylated ZAP-70 complex formed in each aliquot in step e).

11. The method of any of claims 9 or 10, wherein a reduced amount of 7-(4-Aminomethyl-phenylamino)-3-(2,6-dichloro-phenyl)-1-methyl-1 H-[1,6]naphthyridin-2-one - phosphorylated ZAP-70 complex formed in the aliquot incubated with the compound in comparison to the aliquot not incubated with the compound indicates that ZAP-70 is a target of the compound.

12. The method of any of claims 7 to 11, wherein the amount of the 7-(4-Aminomethyl-phenylamino)-3-(2,6-dichloro-phenyl)-1-methyl- 1H-[1,6]naphthyridin-2-one - phosphorylated ZAP-70 complex is determined by separating phosphorylated ZAP-70 from the immobilized 7-(4-Aminomethyl-phenylamino)-3-(2,6-dichloro-phenyl)-1-methyl-1H-[1,6]naphthyridin-2-one and subsequent detection of separated phosphorylated ZAP-70 or subsequent determination of the amount of separated phosphorylated ZAP-70.

13. The method of claim 12, wherein phosphorylated ZAP-70 is detected or the amount of ZAP-70 is determined by mass spectrometry or immunodetection methods, preferably with an antibody directed against phosphorylated ZAP-70, preferably with an antibody recognizing a phosphorylation at position 493 of phosphorylated ZAP-70.

14. The method of any of claims 1 to 13, performed as a medium or high throughput screening.

15. The method of any of claims 1 to 14, wherein said compound is selected from the group consisting of synthetic compounds, or organic synthetic drugs, more preferably small molecule organic drugs, and natural small molecule compounds.

16. The method of any of claims 1 to 15, wherein the ZAP-70 interacting compound is a ZAP-70 inhibitor.

17. The method of any of claims 1 to 16, wherein the solid support is selected from the group consisting of agarose, modified agarose, sepharose beads (e.g. NHS-activated sepharose), latex, cellulose, and ferro- or ferrimagnetic particles.

18. The method of any of claims 1 to 17, wherein the 7-(4-Aminomethyl-phenylamino)-3-(2,6-dichloro-phenyl)-1-methyl-1H-[1,6]naphthyridin-2-one is covalently coupled to the solid support.

19. A method for the purification of phosphorylated ZAP-70, comprising the steps of
a) providing a protein preparation containing phosphorylated ZAP-70,
b) contacting the protein preparation with 7-(4-Aminomethyl-phenylamino)-3-(2,6-dichloro-phenyl)-1-methyl-1H-[1,6]naphthyridin-2-one immobilized on a solid support under conditions allowing the formation of an 7-(4-Aminomethyl-phenylamino)-3-(2,6-dichloro-phenyl)-1-methyl-1H-[1,6]naphthyridin-2-one - phosphorylated ZAP-70 complex, and
c) separating phosphorylated ZAP-70 from the immobilized 7-(4-Aminomethyl-phenylamino)-3-(2,6-dichloro-phenyl)-1-methyl-1H-[1,6]naphthyridin-2-one.

20. The method of claim 19, further comprising after step c) the identification of proteins being capable of binding to phosphorylated ZAP-70.

21. The method of claim 19, further comprising after step c) the determination of post-translational modifications of phosphorylated ZAP-70.

22. The method of any of claims 19 to 21, wherein after step c) either the phosphorylated ZAP-70 and/or the proteins being capable of binding to phosphorylated ZAP-70 are **characterized by** mass spectrometry or immunodetection methods.

23. The method of any of claims 1 to 22, wherein the provision of a protein preparation includes the steps of harvesting at least one cell containing phosphorylated ZAP-70 and lysing the cell.

24. Use of the 7-(4-Aminomethyl-phenylamino)-3-(2,6-dichloro-phenyl)-1-methyl-1H-[1,6]naphthyridin-2-one for the identification of ZAP-70 interacting compounds *in vitro.*

25. Use of the 7-(4-Aminomethyl-phenylamino)-3-(2,6-dichloro-phenyl)-1-methyl-1H-[1,6]naphthyridin-2-one for the purification of phosphorylated ZAP-70.

## Patentansprüche

1. Verfahren für die Identifizierung einer ZAP-70-interagierenden Verbindung, umfassend die Schritte
a) Bereitstellen einer Proteinzubereitung enthaltend phosphoryliertes ZAP-70,
b) Inkontaktbringen der Proteinzubereitung mit 7-(4-Aminomethylphenylamino)-3-(2,6-dichlorphenyl)-1-methyl-1H-[1,6]naphthyridin-2-on, immobilisiert auf einem festen Träger unter Bedingungen, die die Bildung eines 7-(4-Aminomethylphenylamino)-3-(2,6-dichlorphenyl)-1-methyl-1H-[1,6]naphthyridin-2-on - phosphorylierten ZAP-70 Komplexes erlauben,
c) Inkubieren des 7-(4-Aminomethylphenylamino)-3-(2,6-dichlorphenyl)-1-methyl-1H-[1,6]naphthyridin-2-on - phosphorylierten ZAP-70 Komplexes mit einer gegebenen Verbindung, und
d) Bestimmen, ob die Verbindung in der Lage ist, phosphoryliertes ZAP-70 von dem immoblisierten 7-(4-Aminomethylphenylamino)-3-(2,6-dichlorphenyl)-1-methyl-1H-[1,6]naphthyridin-2-on abzutrennen.

2. Verfahren nach Anspruch 1, wobei Schritt d) die Detektion von abgetrenntem, phosphoryliertem ZAP-70 oder die Bestimmung der Menge an abgetrenntem, phosphoryliertem ZAP-70 einschließt.

3. Verfahren nach Anspruch 2, wobei abgetrenntes, phosphoryliertes ZAP-70 detektiert oder die Menge an abgetrenntem, phosporyliertem ZAP-70 durch Massenspektrometrie oder Immundetektionsverfahren bestimmt wird.

4. Verfahren nach Anspruch 3, wobei abgetrenntes, phosphoryliertes ZAP-70 mit einem Antikörper, der gegen phosphoryliertes ZAP-70 gerichtet ist, detektiert wird.

5. Verfahren nach Anspruch 4, wobei abgetrenntes, phosphoryliertes ZAP-70 mit einem Antikörper, der eine Phosphorylierung an Position 493 des phosphorylierten ZAP-70 erkennt, detektiert wird.

6. Verfahren für die Identifizierung einer ZAP-70-interagierenden Verbindung, umfassend die Schritte
a) Bereitstellen einer Proteinzubereitung enthaltend phosphoryliertes ZAP-70,
b) Inkontaktbringen der Proteinzubereitung mit 7-(4-Aminomethylphenylamino)-3-(2,6-dichlorphenyl)-1-methyl-1H-[1,6]naphthyridin-2-on, immobilisiert auf einem festen Träger und mit einer gegebenen Verbindung unter Bedingungen, die die Bildung eines 7-(4-Aminomethylphenylamino)-3-(2,6-dichlorphenyl)-1-methyl-1H-[1,6]naphthyridin-2-on-phosphorylierten ZAP-70 Komplexes erlauben, und
c) Detektieren des in Schritt b) gebildeten 7-(4-Aminomethylphenylamino)-3-(2,6-dichlorphenyl)-1-methyl-1H-[1,6]naphthyridin-2-on - phosphorylierten ZAP-70 Komplexes.

7. Verfahren nach Anspruch 6, wobei in Schritt c) das Detektieren durch Bestimmen der Menge des 7-(4-Aminomethylphenylamino)-3-(2,6-dichlorphenyl)-1-methyl-1H-[1,6]naphthyridin-2-on - phosphorylierten ZAP-70 Komplexes durchgeführt wird.

8. Verfahren nach einem beliebigen der Ansprüche 6 oder 7, wobei die Schritte a) bis c) mit mehreren Proteinzubereitungen durchgeführt werden, um unterschiedliche Verbindungen zu testen.

9. Verfahren für die Identifizierung einer ZAP-70-interagierenden Verbindung, umfassend die Schritte
a) Bereitstellen zweier Aliquots einer Proteinzubereitung enthaltend phosphoryliertes ZAP-70,
b) Inkontaktbringen eines Aliquots mit 7-(4-Aminomethylphenylamino)-3-(2,6-dichlorphenyl)-1-methyl-1H-[1,6]naphthyridin-2-on, immobilisiert auf einem festen Träger unter Bedingungen, die die Bildung eines 7-(4-Aminomethylphenylamino)-3-(2,6-dichlorphenyl)-1-methyl-1H-[1,6]naphthyridin-2-on- phosphorylierten ZAP-70 Komplexes erlauben,
c) Inkontaktbringen des anderen Aliquots mit 7-(4-Aminomethylphenylamino)-3-(2,6-dichlorphenyl)-1-methyl-1H-[1,6]naphthyridin-2-on, immobilisiert auf einem festen Träger und mit einer gegebenen Verbindung unter Bedingungen, die die Bildung eines 7-(4-Aminomethylphenylamino)-3-(2,6-dichlorphenyl)-1-methyl-1H-[1,6]naphthyridin-2-on-phosphorylierten ZAP-70 Komplexes erlauben, und
d) Bestimmen der Menge des in den Schritten b) und c) gebildeten 7-(4-Aminomethylphenylamino)-3-(2,6-dichlorphenyl)-1-methyl-1H-[1,6]naphthyridin-2-on - phosphorylierten ZAP-70 Komplexes.

10. Verfahren für die Identifizierung einer ZAP-70-interagierenden Verbindung, umfassend die Schritte:
a) Bereitstellen zweier Aliquots, von denen jedes mindestens eine Zelle umfasst, die phosphoryliertes ZAP-70 enthält,
b) Inkubieren eines Aliquots mit einer gegebenen Verbindung,
c) Ernten der Zellen eines jeden Aliquots,
d) Lysieren der Zellen, um Proteinzubereitungen zu erhalten,
e) Inkontaktbringen der Proteinzubereitungen mit 7-(4-Aminomethylphenylamino)-3-(2,6-dichlorphenyl)-1-methyl-1H-[1,6]naphthyridin-2-on, immobilisiert auf einem festen Träger unter Bedingungen, die die Bildung eines 7-(4-Aminomethylphenylamino)-3-(2,6-dichlorphenyl)-1-methyl-1H-[1,6]naphthyridin-2-on - phosphorylierten ZAP-70 Komplexes erlauben, und
f) Bestimmen der Menge des in Schritt e) in jedem Aliquot gebildeten 7-(4-Aminomethylphenylamino)-3-(2,6-dichlorphenyl)-1-methyl-1H-[1,6]naphthyridin-2-on - phosphorylierten ZAP-70 Komplexes.

11. Verfahren nach einem beliebigen der Ansprüche 9 oder 10, wobei eine reduzierte Menge des 7-(4-Aminomethylphenylamino)-3-(2,6-dichlorphenyl)-1-methyl-1H-[1,6]naphthyridin-2-on- phosphorylierten ZAP-70 Komplexes, die sich in dem mit der Verbindung inkubierten Aliquot im Vergleich zu dem mit der Verbindung nicht inkubierten Aliquot gebildet hat, darauf hinweist, dass ZAP-70 ein Ziel dieser Verbindung ist.

12. Verfahren nach einem beliebigen der Ansprüche 7 bis 11, wobei die Menge des 7-(4-Aminomethylphenylamino)-3-(2,6-dichlorphenyl)-1-methyl-1H-[1,6]naphthyridin-2-on - phosphorylierten ZAP-70 Komplexes durch Abtrennen des phosphorylierten ZAP-70 von dem immobilisierten 7-(4-Aminomethylphenylamino)-3-(2,6-dichlorphenyl)-1-methyl-1H-[1,6]naphthyridin-2-on und anschließender Detektion des abgetrennten, phosphorylierten ZAP-70 oder anschließender Bestimmung der Menge des abgetrennten, phosphorylierten ZAP-70 bestimmt wird.

13. Verfahren nach Anspruch 12, wobei phosphoryliertes ZAP-70 detektiert wird, oder die Menge an ZAP-70 durch Massenspektometrie oder Immundetektionsverfahren, vorzugsweise mit einem Antikörper, der gegen phosphoryliertes ZAP-70 gerichtet, vorzugsweise mit einem Antikörper, der eine Phosphorylierung an Position 493 des phosphorylierten ZAP-70 erkennt, bestimmt wird.

14. Verfahren nach einem beliebigen der Ansprüche 1 bis 13, durchgeführt als ein Mittel- oder Hochdurchsatzauswahlverfahren.

15. Verfahren nach einem beliebigen der Ansprüche 1 bis 14, wobei die Verbindung ausgewählt ist aus der Gruppe bestehend aus synthetischen Verbindungen oder organisch synthetischen Wirkstoffen, besonders bevorzugt niedermolekularen organischen Wirkstoffen und natürlichen, niedermolekularen Verbindungen.

16. Verfahren nach einem beliebigen der Ansprüche 1 bis 15, wobei die ZAP-70-interagierende Verbindung ein ZAP-70-Inhibitor ist.

17. Verfahren nach einem beliebigen der Ansprüche 1 bis 16, wobei der feste Träger ausgewählt ist aus der Gruppe bestehend aus Agarose, modifizierter Agarose, Sepharose-Beads (z.B. NHS-aktivierte Sepharose), Latex, Cellulose und Ferro- oder ferrimagnetischen Partikeln.

18. Verfahren nach einem beliebigen der Ansprüche 1 bis 17, wobei das 7-(4-Aminomethylphenylamino)-3-(2,6-dichlorphenyl)-1-methyl-1H-[1,6]naphthyridin-2-on kovalent an den festen Träger gekoppelt wird.

19. Verfahren für die Reinigung von phosphoryliertem ZAP-70, umfassend die Schritte
a) Bereitstellen einer Proteinzubereitung enthaltend phosphoryliertes ZAP-70,
b) Inkontaktbringen der Proteinzubereitung mit 7-(4-Aminomethylphenylamino)-3-(2,6-dichlorphenyl)-1-methyl-1H-[1,6]naphthyridin-2-on, immobilisiert auf einem festen Träger unter Bedingungen, die die Bildung eines 7-(4-Aminomethylphenylamino)-3-(2,6-dichlorphenyl)-1-methyl-1H-[1,6]naphthyridin-2-on - phosphorylierten ZAP-70 Komplexes erlauben, und
c) Abtrennen des phosphorylierten ZAP-70 von dem immobilisierten 7-(4-Aminomethylphenylamino)-3-(2,6-dichlorphenyl)-1-methyl-1H-[1,6]naphthyridin-2-on.

20. Verfahren nach Anspruch 19, des Weiteren umfassend nach Schritt c) die Identifizierung von Proteinen, die in der Lage sind, an phosphoryliertes ZAP-70 zu binden.

21. Verfahren nach Anspruch 19, des Weiteren umfassend nach Schritt c) die Bestimmung von post-translationalen Modifikationen von phosphoryliertem ZAP-70.

22. Verfahren nach einem beliebigen der Ansprüche 19 bis 21, wobei nach Schritt c) entweder das phosphorylierte ZAP-70 und/oder die Proteine, die in der Lage sind, an phosphoryliertes ZAP-70 zu binden, durch Massenspektometrie oder Immundetektionsverfahren charakterisiert werden.

23. Verfahren nach einem beliebigen der Ansprüche 1 bis 22, wobei die Bereitstellung einer Proteinzubereitung die Schritte des Emtens mindestens einer Zelle, die phosphoryliertes ZAP-70 enthält, und Lysieren der Zelle einschließt.

24. Verwendung des 7-(4-Aminomethylphenylamino)-3-(2,6-dichlorphenyl)-1-methyl-1H-[1,6]naphthyridin-2-on für die Identifizierung von ZAP-70-interagierenden Verbindungen *in vitro.*

25. Verwendung des 7-(4-Aminomethylphenylamino)-3-(2,6-dichlorphenyl)-1-methyl-1H-[1,6]naphthyridin-2-on für die Reinigung von phosphoryliertem ZAP-70.

## Revendications

1. Procédé d'identification d'un composé d'interaction ZAP-70, comprenant les étapes consistant à
a) fournir une préparation protéinique contenant ZAP-70 phosphorylé,
b) mettre en contact la préparation protéinique avec la 7-(4-aminométhyl-phénylamino)-3-(2,6-dichloro-phényl)-1-méthyl-1H-[1,6]naphtyridin-2-one immobilisée sur un support solide dans des conditions permettant la formation d'un complexe 7-(4-aminométhyl-phénylamino)-3-(2,6-dichloro-phényl)-1-méthyl-1H-[1,6]naphtyridin-2-one/ZAP-70 phosphorylé,
c) incuber le complexe 7-(4-aminométhyl-phénylamino)-3-(2,6-dichlorophényl)-1-méthyl-1H-[1,6]naphtyridin-2-one/ZAP-70 phosphorylé avec un composé donné, et
d) déterminer si le composé est capable de séparer ZAP-70 phosphorylé d'avec la 7-(4-aminométhyl-phénylamino)-3-(2,6-dichlorophényl)-1-méthyl-1H-[1,6]naphtyridin-2-one immobilisée.

2. Procédé selon la revendication 1, dans lequel l'étape d) comprend la détection de ZAP-70 phosphorylé séparé ou la détermination de la quantité de ZAP-70 phosphorylé séparé.

3. Procédé selon la revendication 2, dans lequel ZAP-70 phosphorylé séparé est détecté ou la quantité de ZAP-70 phosphorylé séparée est déterminée par des procédés de spectrométrie de masse ou d'immunodétection.

4. Procédé selon la revendication 3, dans lequel ZAP-70 phosphorylé séparé est détecté par un anticorps dirigé contre ZAP-70 phosphorylé.

5. Procédé selon la revendication 4, dans lequel ZAP-70 phosphorylé séparé est détecté avec un anticorps reconnaissant une phosphorylation de ZAP-70 phosphorylé en position 493.

6. Procédé d'identification d'un composé d'interaction ZAP-70, comprenant les étapes consistant à
a) fournir une préparation protéinique contenant ZAP-70 phosphorylé,
b) mettre en contact la préparation protéinique avec la 7-(4-aminométhyl-phénylamino)-3-(2,6-dichloro-phényl)-1-méthyl-1H-[1,6]naphtyridin-2-one immobilisée sur un support solide et avec un composé donné dans des conditions permettant la formation d'un complexe 7-(4-aminométhyl-phénylamino)-3-(2,6-dichloro-phényl)-1-méthyl-1H-[1,6]naphtyridin-2-one/ZAP-70 phosphorylé,
c) détecter le complexe 7-(4-aminométhyl-phénylamino)-3-(2,6-dichloro-phényl)-1-méthyl-1H-[1,6]naphtyridin-2-one/ZAP-70 phosphorylé formé à l'étape b).

7. Procédé selon la revendication 6, dans lequel à l'étape c) ladite détection est réalisée en déterminant la quantité de complexe 7-(4-aminométhyl-phénylamino)-3-(2,6-dichlorophényl)-1-méthyl-1H-[1,6]naphtyridin-2-one/ZAP-70 phosphorylé.

8. Procédé selon l'une quelconque des revendications 6 ou 7, dans lequel les étapes a) à c) sont réalisées avec plusieurs préparations protéiniques afin d'évaluer différents composés.

9. Procédé d'identification d'un composé d'interaction ZAP-70, comprenant les étapes consistant à :
a) fournir deux fractions aliquotes d'une préparation protéinique contenant ZAP-70 phosphorylé,
b) mettre en contact une fraction aliquote avec la 7-(4-aminométhyl-phénylamino)-3-(2,6-dichloro-phényl)-1-méthyl-1H-[1,6]naphtyridin-2-one immobilisée sur un support solide dans des conditions permettant la formation d'un complexe 7-(4-aminométhyl-phénylamino)-3-(2,6-dichloro-phényl)-1-méthyl-1H-[1,6]naphtyridin-2-one/ZAP-70 phosphorylé,
c) mettre en contact l'autre fraction aliquote avec la 7-(4-aminométhyl-phénylamino)-3-(2,6-dichloro-phényl)-1-méthyl-1H-[1,6]naphtyridin-2-one immobilisée sur un support solide et avec un composé donné dans des conditions permettant la formation d'un complexe 7-(4-aminométhyl-phénylamino)-3-(2,6-dichloro-phényl)-1-méthyl-1H-[1,6]naphtyridin-2-one/ZAP-70 phosphorylé,
d) déterminer la quantité de complexe 7-(4-aminométhyl-phénylamino)-3-(2,6-dichloro-phényl)-1-méthyl-1H-[1,6]naphtyridin-2-one/ZAP-70 phosphorylé formé aux étapes b) et c).

10. Procédé d'identification d'un composé d'interaction ZAP-70, comprenant les étapes consistant à :
a) fournir deux fractions aliquotes comprenant chacune au moins une cellule contenant ZAP-70 phosphorylé,
b) incuber une fraction aliquote avec un composé donné,
c) recueillir les cellules de chaque fraction aliquote,
d) lyser les cellules afin d'obtenir des préparations protéiniques,
e) mettre en contact les préparations protéiniques avec la 7-(4-aminométhyl-phénylamino)-3-(2,6-dichloro-phényl)-1-méthyl-1H-[1,6]naphtyridin-2-one immobilisée sur un support solide dans des conditions permettant la formation d'un complexe 7-(4-aminométhyl-phénylamino)-3-(2,6-dichloro-phényl)-1-méthyl-1 H-[1,6]naphtyridin-2-one/ZAP-70 phosphorylé, et
f) déterminer la quantité de complexe 7-(4-aminométhyl-phénylamino)-3-(2,6-dichloro-phényl)-1-méthyl-1H-[1,6]naphtyridin-2-one/ZAP-70 phosphorylé formé dans chaque fraction aliquote de l'étape e).

11. Procédé selon l'une quelconque des revendications 9 ou 10, dans lequel une quantité réduite du complexe 7-(4-aminométhyl-phénylamino)-3-(2,6-dichloro-phényl)-1-méthyl-1H-[1,6]naphtyridin-2-one/ZAP-70 phosphorylé formé dans la fraction aliquote incubée avec le composé en comparaison avec la fraction aliquote non incubée avec le composé indique que ZAP-70 est une cible du composé.

12. Procédé selon l'une quelconque des revendications 7 à 11, dans lequel la quantité du complexe 7-(4-aminométhyl-phénylamino)-3-(2,6-dichloro-phényl)-1-méthyl-1H-[1,6]naphtyridin-2-one/ZAP-70 phosphorylé est déterminée en séparant ZAP-70 phosphorylé d'avec la 7-(4-aminométhyl-phénylamino)-3-(2,6-dichloro-phényl)-1-méthyl-1H-[1,6]naphtyridin-2-one immobilisée et la détection ultérieure de ZAP-70 phosphorylé séparé ou la détermination ultérieure de la quantité de ZAP-70 phosphorylé séparé.

13. Procédé selon la revendication 12, dans lequel ZAP-70 phosphorylé est détecté ou la quantité de ZAP-70 est déterminée par des procédés de spectrométrie de masse ou d'immunodétection, de préférence avec un anticorps dirigé contre ZAP-70 phosphorylé, de préférence avec un anticorps reconnaissant une phosphorylation en position 493 de ZAP-70 phosphorylé.

14. Procédé selon l'une quelconque des revendications 1 à 13, réalisé sous la forme d'un criblage à débit moyen ou élevé.

15. Procédé selon l'une quelconque des revendications 1 à 14, dans lequel ledit composé est choisi dans le groupe constitué par des composés synthétiques ou des médicaments synthétiques organiques, de manière plus particulièrement préférée des médicaments organiques à petites molécules et des composés naturels à petites molécules.

16. Procédé selon l'une quelconque des revendications 1 à 15, dans lequel le composé d'interaction ZAP-70 est un inhibiteur ZAP-70.

17. Procédé selon l'une quelconque des revendications 1 à 16, dans lequel le support solide est choisi dans le groupe constitué par l'agarose, l'agarose modifié, les perles de Sepharose (par exemple la Sepharose activée au NHS), le latex, la cellulose et les particules ferro- ou ferrimagnétiques.

18. Procédé selon l'une quelconque des revendications 1 à 17, dans lequel la 7-(4-aminométhyl-phénylamino)-3-(2,6-dichloro-phényl)-1-méthyle-1H-[1,6]-naphtyridin-2-one est liée de manière covalente au support solide.

19. Procédé de purification de ZAP-70 phosphorylé, comprenant les étapes consistant à
a) fournir une préparation protéinique contenant ZAP-70 phosphorylé,
b) mettre en contact la préparation protéinique avec la 7-(4-aminométhyl-phénylamino)-3-(2,6-dichloro-phényl)-1-méthyl-1H-[1,6]naphtyridin-2-one immobilisée sur un support solide dans des conditions permettant la formation d'un complexe 7-(4-aminométhyl-phénylamino)-3-(2,6-dichloro-phényl)-1-méthyl-1H-[1,6]naphtyridin-2-one/ZAP-70 phosphorylé, et
c) séparer ZAP-70 phosphorylé d'avec la 7-(4-aminométhyl-phénylamino)-3-(2,6-dichloro-phényl)-1-méthyl-1H-[1,6]naphtyridin-2-one immobilisée.

20. Procédé selon la revendication 19, comprenant en outre après l'étape c) l'identification de protéines capables de se lier à ZAP-70 phosphorylé.

21. Procédé selon la revendication 19, comprenant en plus après l'étape c) la détermination de modifications post-traductionnelles de ZAP-70 phosphorylé.

22. Procédé selon l'une quelconque des revendications 19 à 21, dans lequel après l'étape c) ZAP-70 phosphorylé et/ou les protéines capables de se lier à ZAP-70 phosphorylé sont **caractérisés par** des procédés de spectrométrie de masse ou d'immunodétection.

23. Procédé selon l'une quelconque des revendications 1 à 22, dans lequel la fourniture d'une préparation protéinique comprend les étapes consistant à recueillir au moins une cellule contenant ZAP-70 phosphorylé et à lyser la cellule.

24. Utilisation de la 7-(4-aminométhyl-phénylamino)-3-(2,6-dichloro-phényl)-1-méthyl-1H-[1,6]naphtyridin-2-one pour l'identification *in vitro* des composés d'interaction de ZAP-70.

25. Utilisation de la 7-(4-aminométhyl-phénylamino)-3-(2,6-dichloro-phényl)-1-méthyl-1H-[1,6]naphtyridin-2-one pour la purification de ZAP-70 phosphorylé.
